# EUROPEAN PATENT APPLICATION

(11) **EP 4 462 105 A1**
(43) Date of publication of application: **13.11.2024**
(21) Application number: 23382435.8
(22) Date of filing: 10.05.2023
(51) Int. Cl.: G01N 21/65

(54) **SERS-ACTIVE REAGENT, METHOD OF SYNTHESIZING THE SERS-ACTIVE REAGENT, AND METHOD OF DETECTING AN ANALYTE IN A SAMPLE USING THE SERS-ACTIVE REAGENT**

(71) Applicant: MEDCOM ADVANCE, S.A., 08029 Barcelona (ES)
(72) Inventor: GÓMEZ DE PEDRO, Sara, E-43007 Tarragona (ES); GARCÍA ALGAR, Manuel, E-43007 Tarragona (ES); MUR SUÑÉ, Anna, E-43007 Tarragona (ES); RIVERO GARCÍA, María, E-43007 Tarragona (ES); CAPARRÓS RODRÍGUEZ, Francisco Javier, E-43007 Tarragona (ES); SAGALÉS MAÑAS, Juan, E-08840 Viladecans, Barcelona (ES)
(74) Representative: ABG Intellectual Property Law, S.L.

(57) **Abstract**

A SERS-active reagent is described, the SERS-active reagent comprising, in order:

a) a core of a particle functionalized for binding of element b);

b) a shell of a plurality of nanoparticles of a plasmonic material immobilized on the surface of the particle a), wherein the surface of the nanoparticles is coated with aliphatic, aromatic and/or polymeric molecules or combinations thereof, and at least one Raman label,

c) at least one layer of a coating material covering element b) that interacts with element d); optionally, wherein the layer of a coating material is functionalized with a compound that allows the immobilization of a reagent capable of detecting an analyte; and

d) a reagent immobilized on the coating layer c) capable of detecting an analyte.

## Description

### FIELD OF THE INVENTION

The invention relates to the field of diagnostic methods.

### BACKGROUND OF THE INVENTION

Pathogen infection is a serious concern in human health. Specifically, sepsis, a major complication of the body to infections, is one of the most threats to public health.

In terms of medical care, sepsis is a global priority, affecting around 50 million people worldwide every year, and increasing annually. This complication has a high mortality rate, estimated in at least 11 million deaths per year, ranking third, even behind of prostate cancer, breast cancer or acquired immunodeficiency syndrome combined cases. Depending on the country, mortality varies between 15 and more than 50%. Additionally, many patients who survive to sepsis have severe consequences for the rest of their lives. Costs associated to the health system must also be considered, which include not only the cost of diagnosis and treatment, but also those related to hospitalization (including Intensive Care Unit), being of up to 24 million dollars only in the United States (Global Sepsis Alliance. May 2023) (Cockerill F, Wilson J, Vetter E, Goodman K, Torgerson C, Harmsen W, et al. Clin Infec Dis. 38, 1724 (2004)) (World Health Organization. WHO Report on Sepsis. April 2018).

Early detection of sepsis is of crucial importance, since the chance of survival are closely dependent on the administration of the appropriate treatment. In general, it is considered that for each hour of delay in the antibiotic or antifungal treatment, the chance for survival decreases in approximately an 8%, and, without therapy, the chance for survival is less than 50% in the first 4 hours after septic shock. In addition, the use of specific therapy reduces mortality between 40 and 80% depending on the severity of sepsis. On the contrary, the survival rate drops to 10% if the patient has not taken specific therapy during the first 24 hours. Cases of recurrence and re-hospitalization must also be taken into account, with a 19% of incidence in the first 30 days (Angus D C, van der Poll T, N Engl J Med. 369, 840 (2013), (Kumar A. et al., Crit. Care Med. 34, 1589 (2006)), (Hotchkiss R, Moldawer L, Opal S, et al. Nat Rev Dis Primers. 2, 16045 (2016)).

Sepsis diagnosis is based on microbiological tests, being blood culture the reference standard. To do so, 40-60 ml of blood are taken from the patient and cultured under aerobic and anaerobic conditions. Positive confirmation takes a minimum of 10-12 hours, increasing up to 24 hours in some cases. Gram staining is performed on positive blood cultures to make a preliminary identification of the pathogen (morphology; Gram +/-). In parallel, the specific identification of the pathogen is performed through MALDI-ToF mass spectrometry, which takes between 30-60 minutes, or through an additional subculture in specific media, which can take between 12-24 hours and up to 14 days in those microorganisms with slower growth. Despite the fact that clinical microbiology laboratories are implementing advances for automation, the current reference method of pathogen identification for sepsis has certain limitations, such as the case of non-culturable microorganisms or when antibiotic treatment has been administered prior to sampling, which inhibits or retards microbial growth and thus prevents identification of organisms based on blood cultures (Fournier PE, Drancourt M, Colson P, et al. Nat Rev Microbiol. 11, 574 (2013)).

In recent years, several proposals have emerged as an alternative to blood cultures. The most important and accepted on the market are ELISA (Enzyme-Linked Immuno Sorbent Assay) and its modalities, PCR (Polymerase Chain Reaction), or MALDI-ToF (Matrix-Assisted Laser Desorption/lonization -Time -of-Flight) (Kothari A, et al. Clinical infectious diseases: an official publication of the Infectious Diseases Society of America. 59, 2, 272 (2014)), (Peters RPH, et al. Lancet Infect. Dis. 4, 12, 751 (2004)).

Systems based on immunological technologies can be quite fast and simple, can become portable, and usually require small sample volumes. However, they are normally limited by their low sensitivity and normally are not multiplex systems. (Brunauer A, Verboket RD, Kainz DM, von Stetten F, Früh SM. Biosens. 11, 3, 74 (2021).)(Ha Y, Kim I, BioChip J. 16, 351 (2022)), (Rudenko, NV, et al. J Food Drug Anal. 26, 2, 741 (2018)).

Molecular diagnostic techniques such as PCR detect specific genes, and usually provide results in 4-8 hours. However, these techniques have their limitations in terms of blood analysis, since they have interferences due to the presence of free DNA in blood. Furthermore, these tests require the lysing of the bacteria rather than the detection of the pathogen itself (Opota O, Jaton K, Greub G, Clin. Microbiol. Infect. 21, 4, 323 (2015)), (Garibyan L, Nidhi A. J Invest Dermatol. 133, 3, 1 (2013)), (Zemanick ET, Wagner BD, Sagel SD, Stevens MJ, Accurso FJ, et al. PLOS ONE 5(11), e15101 (2010)).

MALDI-ToF technology confers high selectivity and sensitivity, but also requires bacterial blood culture. Although this method is multiplex, it is still slow as it requires the culture and the isolation of the agent to be analyzed (24-72h), it is not portable, and it is considerably expensive, requiring a frequent maintenance. (Dixon P, et al. Eur J Clin Microbiol Infec. 34, 5, 863 (2015)), (Morgenthaler NG, Kostrzewa M, Int J Microbiol. ID 827416 (2015)), (Edith BDW, Zemanick T, Sagel SD, Stevens MJ, Accurso FJ, Harris JK, PLoS One, 5, e15101 (2010)).On the other hand, great advances are being made in magnetic resonance imaging systems (T2MR). The T2Bacteria^{®} Panel developed by the company T2Biosystems, is the first and only test approved by the FDA and with the CE marking to identify bacteria that cause sepsis directly from whole blood without blood culture. The system appears promising, since it allows the identification in 3 to 5 hours, however, it does not detect all types of infections. Thus, identifying the causative pathogen is one of the priorities for sepsis diagnosis and treatment, where diagnostic methods must be fast and accurate for the patient healing (Kalligeros, M, et al. BMC Infect Dis, 20,1, 326, 2020), (Muñoz P, Vena A, Machado M, et al. J. Antimicrob. Chemother. 73(suppl_4):iv13-iv19 (2018)).

The need to improve the sensitivity, specificity and/or analysis time of the current methods for sepsis microorganism identification is clear.

### SUMMARY OF THE INVENTION

In a first aspect, the invention relates to a surface-enhanced Raman scattering-(SERS)-active reagent, comprising in order:
a) a core of a particle functionalized for binding of element b);
b) a shell of a plurality of nanoparticles of a plasmonic material immobilized on the surface of the particle a) wherein the surface of the nanoparticles is coated with aliphatic, aromatic and/or polymeric molecules or combinations thereof and at least one Raman label,
c) at least one layer of a coating material covering element b) that interacts with element d); optionally, the layer of the coating material is functionalized with a compound that allows the immobilization of a reagent capable of detecting an analyte; and
d) a reagent immobilized on the coating layer c) capable of detecting an analyte.

In another aspect, the invention relates to a method for detecting and identifying the presence and/or the amount of at least one analyte in a sample, the method comprising:
a) incubating the sample comprising the analyte with a reagent capable of recognizing the analyte, during a time sufficient to allow the formation of a complex between the reagent and the analyte;
b) separating the complex of the reagent and the analyte from the sample;
c) optionally, staining the analyte with a fluorescence label;
d) eluting the analyte from the complex
e) incubating the eluted analyte with at least one SERS reagent according to the invention during a time sufficient to allow the labelling of the eluted analyte with the SERS reagent; and
f) acquiring an image of the sample with an optical and/or fluorescence microscope and
g) measuring Raman spectroscopy to detect and identify the presence and/or amount of the at least one analyte.

In another aspect, the invention relates to a method for synthesizing the reagent of the invention comprising the steps of:
a) providing particles functionalized for binding nanoparticles of step b);
b) adding nanoparticles of a plasmonic material and allowing the immobilization of a plurality of nanoparticles on the surface of the particles of step a) to form a cluster, wherein the surface of the nanoparticles is coated with aliphatic, aromatic and/or polymeric molecules or combinations thereof and at least one Raman label
c) covering the surface of the cluster with at least one layer of a coating material;
d) optionally, functionalizing the coated material with a compound that allows the immobilization of a reagent capable of detecting an analyte; and
e) adding a reagent capable of detecting an analyte to allow the binding of the reagent to the coated cluster.

In another aspect, the invention relates to the use of the surface-enhanced Raman scattering-(SERS)-active reagent according to the invention in a method for detecting, identifying and/or quantifying an analyte.

### DESCRIPTION OF THE FIGURES

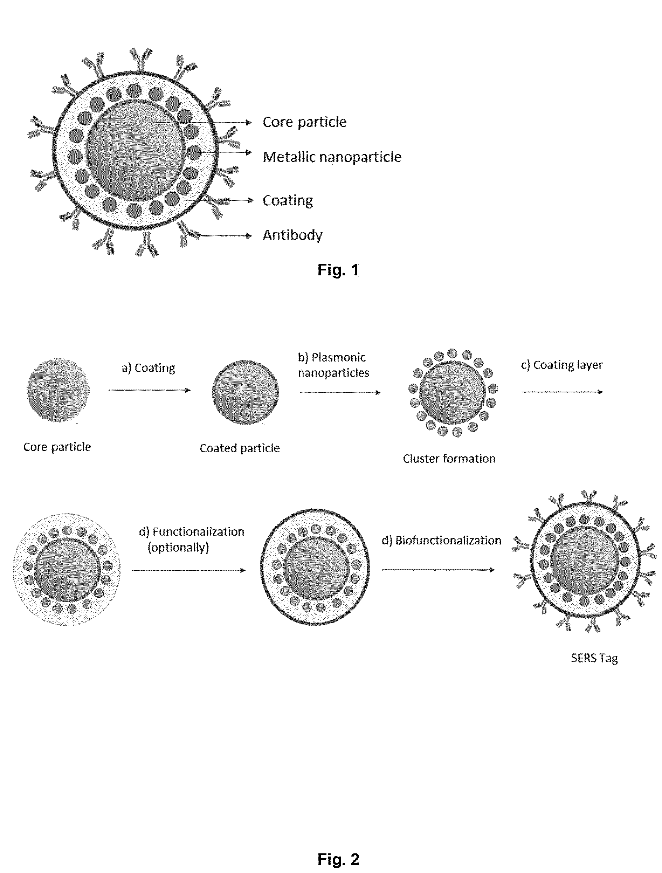

**Figure 1****.** Schematic representation of an example of core particle covered with metallic nanoparticles and coated with an uniform layer, which is biofunctionalized with a biorecognition element, such as antibodies.
**Figure 2****.** Schematic representation of the synthetic procedure of the SERS Tags. Example with antibody biofunctionalization.

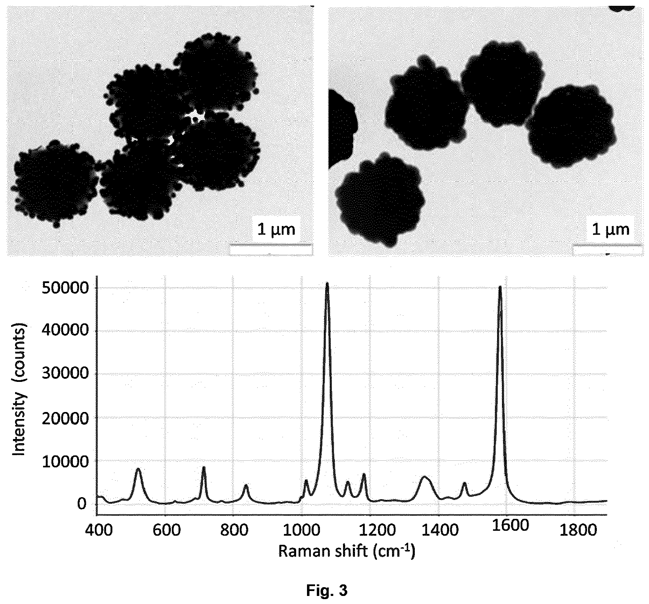

**Figure 3****.** Example of SERS Tags, with a 1 µm magnetic core microparticles, conjugated with 70 nm silver nanoparticles and a silica layer, biofunctionalized with a C. *albicans* selective antibody. In the top, from left to right: TEM image of the conjugated particles, previous to silica coating; TEM image of the same particles once silica-coated and biofunctionalized with the antibody (JEOL 1011, 100kV); In the bottom, Raman spectrum (RA816 Renishaw plc) of a single magnetic SERS Tag, in this case, functionalized with 4-mercaptobenzoic acid (4-MBA) as Raman Label.

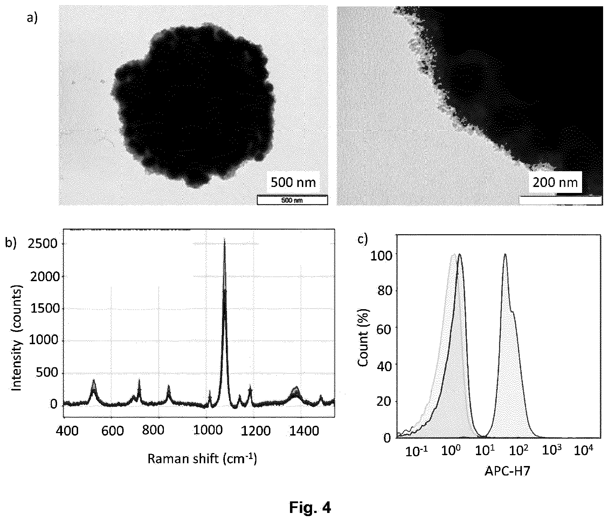

**Figure 4****.** Example of SERS Tags, with a 1 µm magnetic core microparticle conjugated with 70 nm silver nanoparticles and a titania layer, biofunctionalized with a C. *albicans* selective antibody. a) TEM images (JEOL 1011, 100kV) from the titania coated particles; b) Raman spectra (RA816 Renishaw plc) of the magnetic SERS Tags, in this case, functionalized with 4-mercaptobenzoic acid (4-MBA) as Raman Label. The spectra show signal providing from 24 different single particles. c) Flow cytometer plot from the biofunctionalization (APC filter, exc 651 nm, ems 660 nm). Left bands correspond to negative controls, obtained from non-conjugated SERS Tags and conjugated the SERS Tags with an anti-mouse from goat secondary antibody, respectively. The righter band corresponds to the positive SERS Tags sample, obtained from incubating Tags with an anti-rabbit from goat secondary antibody.

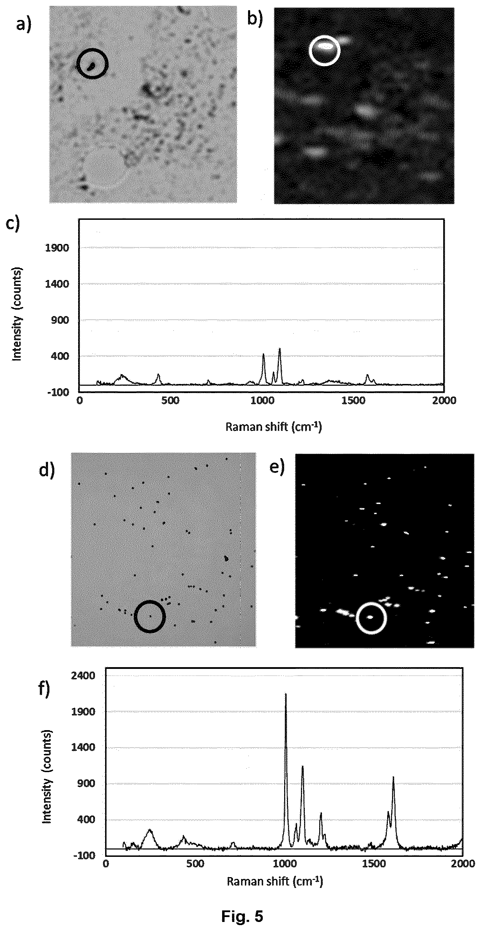

**Figure 5****.** Raman signal comparison of clustered silver nanoparticles, where hot spots are present, and the developed SERS Tags. a) White light image of the cluster area of nanoparticles. b) Raman map of the registered area for the cluster of nanoparticles. c) Raman spectrum of the selected area of the cluster of nanoparticles, marked in the images with a circle. d) White light image of the SERS Tags. e) Raman map for the SERS Tags. f) Raman spectrum of the selected SERS Tag, marked in the images with a circle. As it can be observed, intensity is much higher in case of the developed SERS reagent.

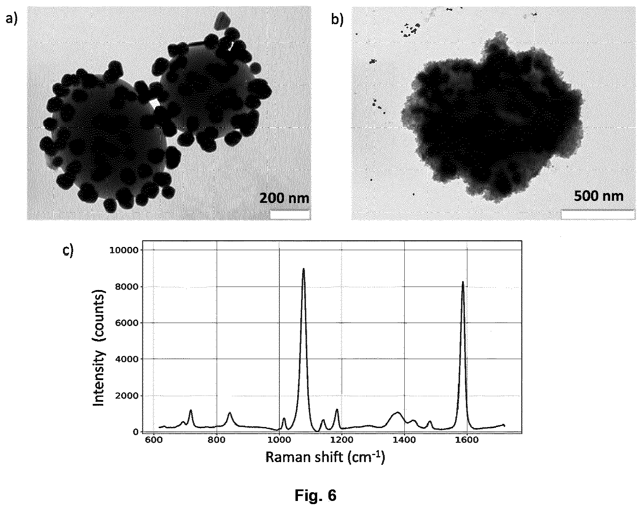

**Figure 6****.** Example of SERS Tags with a polystyrene core microparticle of 0.5 µm. a) TEM image from conjugated silver nanoparticles of 70 nm, functionalized with 4-MBA as Raman label. b) TEM image of same particles with a titania coating. c) Raman spectra (RA816 Renishaw plc) of the SERS Tags functionalized 4-MBA. The spectrum is the average from 24 different spectra of single particles.

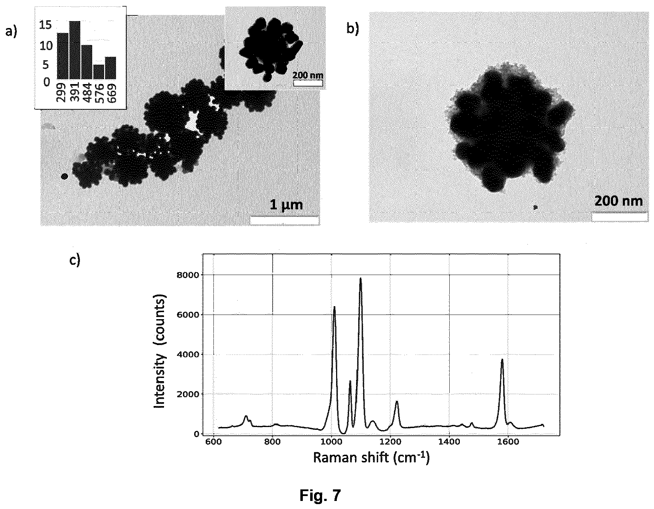

**Figure 7****.** Example of magnetic SERS Tags with an average core size microparticle of 0.45 µm. a) TEM image from conjugated silver nanoparticles of 70 nm, functionalized with 4-MPy as Raman label. Size distribution of the core microparticles can be seen in the image as well as in the histogram. A TEM amplification with a single particle is also shown. b) TEM image of same particles with a titania coating. c) Raman spectra (RA816 Renishaw plc) of the SERS Tags functionalized 4-MPy. The spectrum is the average from 24 different spectra of single particles.

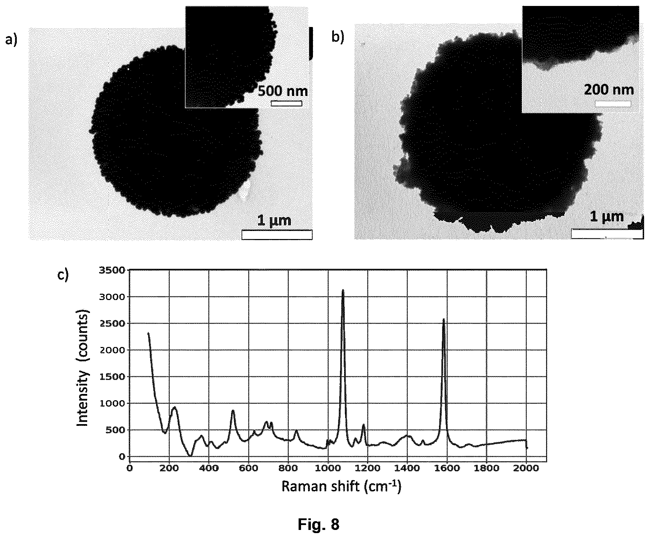

**Figure 8****.** Example of magnetic SERS Tags with an average core size microparticle of 2.8 µm. a) TEM image from conjugated silver nanoparticles of 70 nm, functionalized with 4-MBA as Raman label. b) TEM image of same particles with a titania coating. TEM amplification images are shown in both cases. c) Raman spectra (RA816 Renishaw plc) of the SERS Tags functionalized 4-MBA. The spectrum is the average from 24 different spectra of single particles.

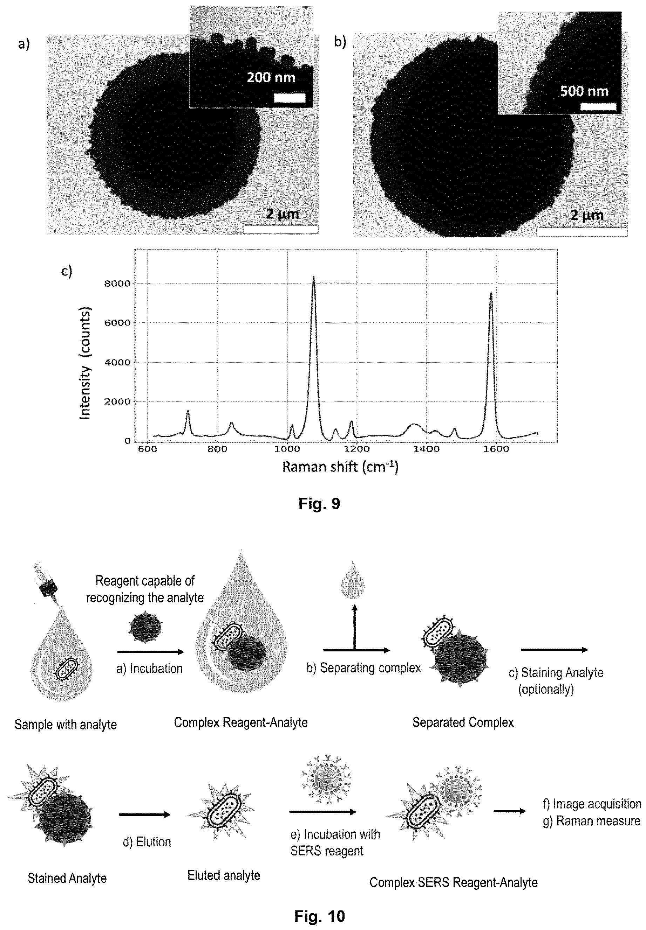

**Figure 9****.** Example of magnetic SERS Tags with an average core size microparticle of 4.5µm. a) TEM image from conjugated silver nanoparticles of 70 nm, functionalized with 4-MBA as Raman label. b) TEM image of same particles with a titania coating. TEM amplification images are shown in both cases. c) Raman spectra (RA816 Renishaw plc) of the SERS Tags functionalized 4-MBA. The spectrum is the average from 24 different spectra of single particles.
**Figure 10****.** Schematic representation of the method for detecting and identifying the presence and/or the amount of at least one analyte in a sample, such as the detection of microorganisms in biofluids.

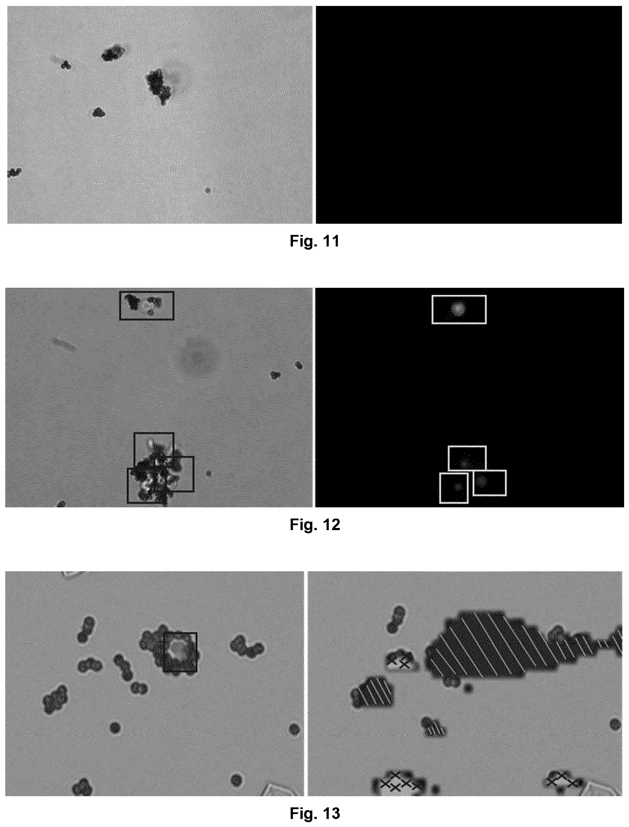

**Figure 11****.** White light and fluorescence images (left and right) of the blank sample.
**Figure 12****.** White light and fluorescence images (left and right) of the sample with *C. albicans.* Squares label where the microorganism is presented.
**Figure 13****.** White light images of a sample (left), and same images with SERS signal overlapped (right) from two different SERS Tags. The microorganism, *Candida,* is labelled with a square. SERS intensities are highlighted with solid grey shapes, where the dark shape with lines corresponds to one type of SERS Tag (synthesized with a Raman Label 1, 4-MPy (4-Mercaptopyridine), and an antibody selective to *C. albicans*), and the light shape with crosses corresponds to the second type of SERS Tag (synthesized with a Raman Label 2, 4-MBA, and an antibody selective to *S. aureus*).

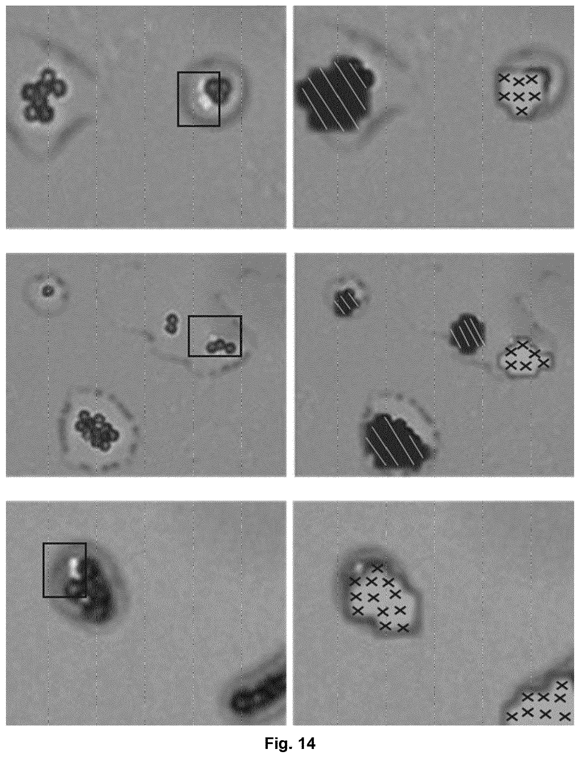

**Figure 14****.** White light images of the sample (left), and same images with overlapped signals from two different RL (right). The microorganism, *S. aureus,* is labelled with a square. SERS intensities are highlighted with solid grey shapes, where the dark shape with lines corresponds to one type of SERS Tag (synthesized with a Raman Label 1, 4-MPy, and an antibody selective to *C. albicans*), and the light shape with crosses corresponds to the second type of SERS Tag (synthesized with a Raman Label 2, 4-MBA, and an antibody selective to *S. aureus*).

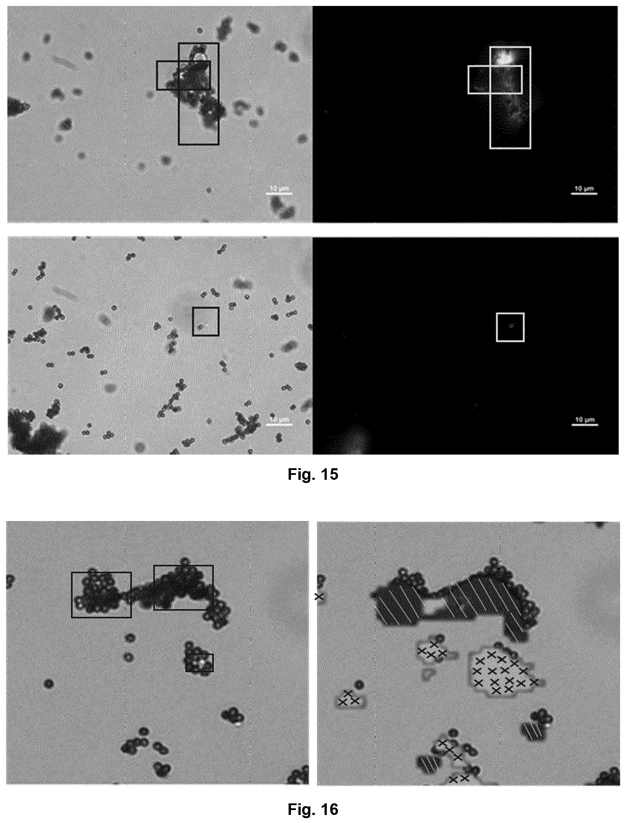

**Figure 15****.** White light and fluorescence images (left and right) of a sample with two different microorganisms. On the top, images show the presence of *C. albicans* fungi. On the bottom, images show the presence of bacterium S. *aureus.* Squares label where the microorganism is presented.
**Figure 16****.** Images of a sample with two different microorganisms, in white light (left) and same images with the SERS signal overlapped (right) for two different SERS Tags. The microorganisms, *C. albicans* and *S. aureus,* are labelled with a square. SERS intensities are highlighted with solid grey shapes, where the dark shape with lines corresponds to one type of SERS Tag (synthesized with a Raman Label 1, 4-MPy, and an antibody selective to *C. albicans*), and the light shape with crosses corresponds to the second type of SERS Tag (synthesized with a Raman Label 2, 4-MBA, and an antibody selective to S. *aureus*).

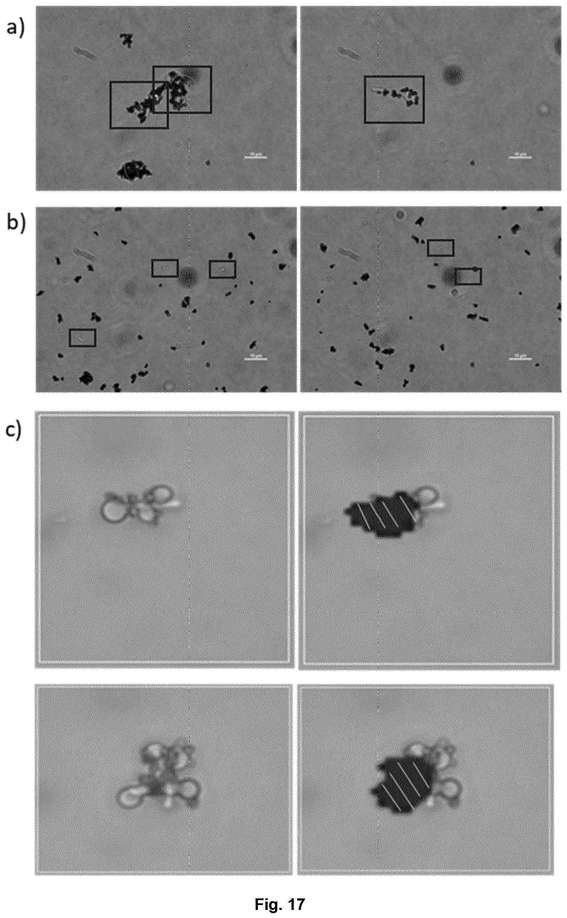

**Figure 17****.** Use of magnetic SERS Tags of 0.45 µm core diameter, with 4-MPy as Raman label, titania coated and with a selective antibody to *C. albicans.* a) Identification of *C. albicans.* b) Cross-reactivity with *S. aureus.* c) White light image and overlapping of white light image with SERS mapping for the magnetic SERS particles attached to fungi. Fungi and bacteria are labelled with a square. SERS intensity is highlighted in dark grey with lines (c).

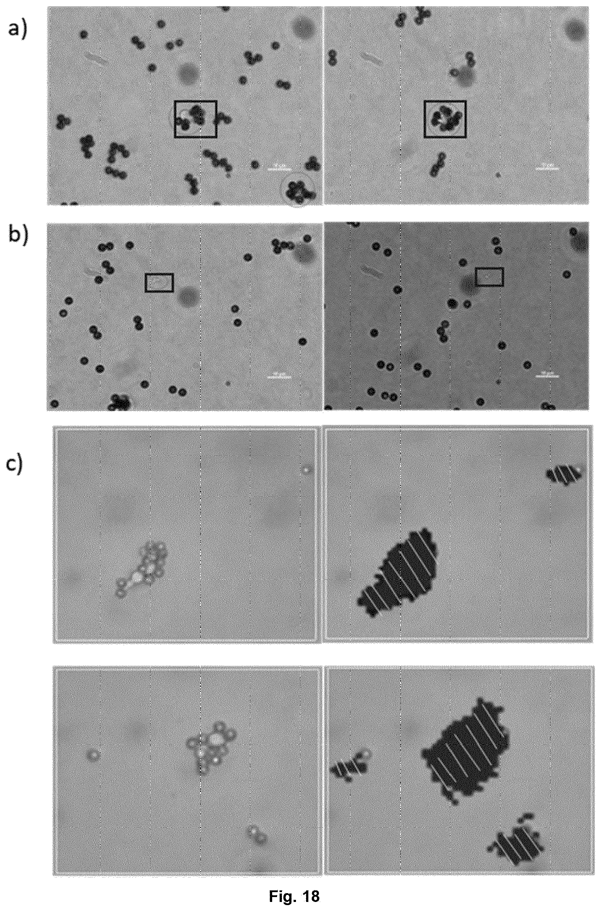

**Figure 18****.** Use of magnetic SERS Tags of 2.8 µm core diameter, with 4-MPy as Raman label, titania coated and with a selective antibody to *C. albicans.* a) Identification of *C. albicans.* b) Cross-reactivity with *S. aureus.* c) White light image and overlapping of white light image with SERS mapping for the magnetic SERS particles attached to fungi. Fungi and bacteria are labelled with a square. SERS intensity is highlighted in dark grey with lines (c).

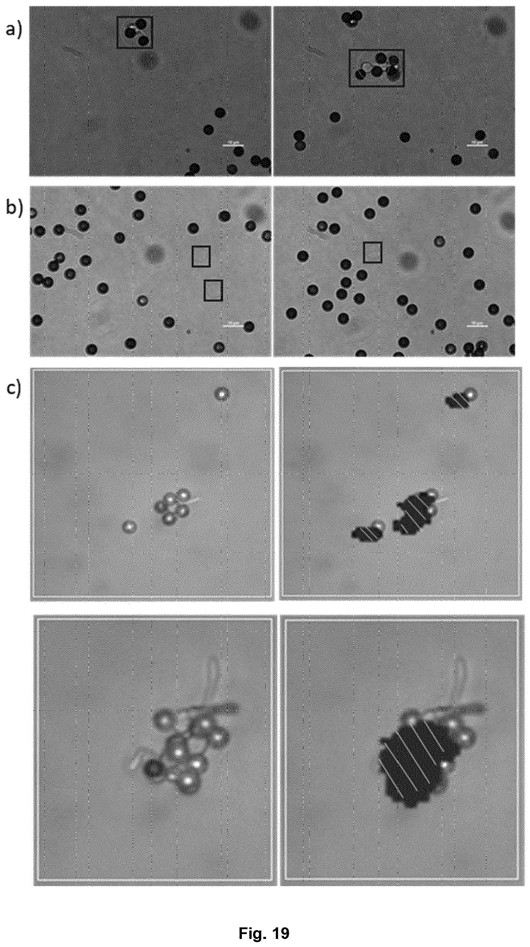

**Figure 19****.** Use of magnetic SERS Tags of 4.5 µm core diameter, with 4-MPy as Raman label, titania coated and with a selective antibody to *C. albicans.* a) Identification of *C. albicans.* b) Cross-reactivity with *S. aureus.* c) White light image and overlapping of white light image with SERS mapping for the magnetic SERS particles attached to fungi. Fungi and bacteria are labelled with a square. SERS intensity is highlighted in dark grey with lines (c).

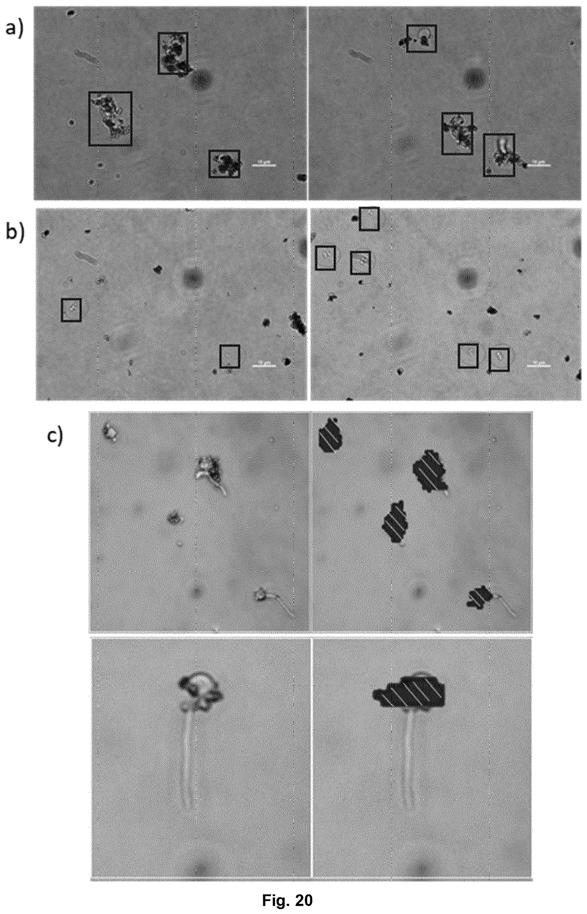

**Figure 20****.** Use of polystyrene SERS Tags of 0.5 µm core diameter, with 4-MPy as Raman label, titania coated and with a selective antibody to *C. albicans.* a) Identification of *C. albicans.* b) Cross-reactivity with *S. aureus.* c) White light image and overlapping of white light image with SERS mapping for the magnetic SERS particles attached to fungi. Fungi and bacteria are labelled with a square. SERS intensity is highlighted in dark grey with lines (c).

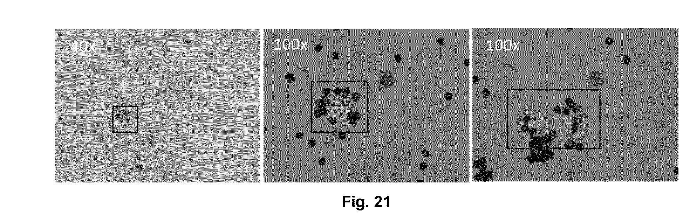

**Figure 21****.** Use of 2.8 µm diameter magnetic SERS Tags, with 4-MPy as Raman label, titania coated and with a selective antibody to HER2. White light images show SERS Tags are attached to AU565 cells, demonstrating its selective identification. Cells are labelled with a square.

### DESCRIPTION OF THE INVENTION

The authors of the present invention have developed a reagent for the detection and identification of analytes such as microorganism and more specifically, bacteria and fungi in blood samples.

The invention includes a biochemical assay based on a specially designed reagent, and the use of spectroscopy as analytical technique. It is a fast method, since results are provided in 4-5 hours, including the biochemical assay and the final spectroscopic measurement. In a particular example, results of the method provide sepsis diagnosis including the identification and quantification of the causative microorganism. It is worth to mention that the method could be applied to the identification of other analytes, such as circulating tumour cells.

### Reagent of the invention

SERS is a well-known ultrasensitive analytical technique which can provide single molecule detection by improving the efficiency of Raman spectroscopy through the Raman enhancement produced when molecules are adsorbed on the surface of noble metal nanomaterials, such as nanoparticles. In essence, the SERS tags employed in the developed assay include nanoparticles with plasmonic nucleus, which generates the electric field necessary for the Raman signal amplification of the molecules adsorbed on its surface. Furthermore, when nanoparticles are close together, coupling of individual electric fields is produced, changing the extinction profile. This additional enhancement has great effect on the SERS signal as it can be increased several orders of magnitude (>10³ times). Depending on the material and the size of the nanoparticles, this effect can be maximized. These two effects are responsible for the strong SERS signal of the developed SERS tags.

The inventors have developed a SERS reagent (also indicated as SERS-tag or SERS Tag) comprising a plasmonic nucleus, responsible for the generation of the electric field necessary for the Raman amplification; a Raman probe (i.e. code, label), responsible for the unique vibrational fingerprint of the encoded particle; and a coating layer. The reagent of the invention has several advantages. Nanoparticles make possible the identification of the analyte, correlating the obtained signal in the Raman spectroscopy measurement with, for example, the antibody immobilized on the surface of the cluster. Since nanoparticles of a plasmonic material can be functionalized with different molecules which show a specific Raman signal (Raman markers, Raman labels, RL), a collection of SERS Tags with different signals can be obtained. It is possible then to identify for example a pathogen which is causing the infection according to the RL detected in the spectroscopic measurement. Another advantage of the reagent is the intrinsic intense and stable SERS signal of the SERS Tags, which allows high optical efficiency and confident measurements. SERS efficiency of the reagent is much higher than clustered plasmonic nanoparticles. This is due to the stable and homogeneous shell of plasmonic nanoparticles. The external coating layer can also improve the stability of the cluster. Thus, the developed SERS Tag can be also stable in different media conditions, is photostable, and has low toxicity, enlarging the possible applications of the reagent. It is worth to mention that size of the reagents can be tuned depending on the core of the particle need for the specific application. All these advantages make feasible a fast analysis of samples.

Thus, in a first aspect, the invention relates to a surface-enhanced Raman scattering-(SERS)-active reagent, comprising in order:
a) a core of a particle functionalized for binding of element b);
b) a shell of a plurality of nanoparticles of a plasmonic material immobilized on the surface of the particle a) wherein the surface of the nanoparticles is coated with aliphatic, aromatic and/or polymeric molecules or combinations thereof, and at least one Raman label,
c) at least one layer of a coating material covering element b) that interacts with element d); optionally, the layer of the coating material is functionalized with a compound that allows the immobilization of a reagent capable of detecting an analyte; and
d) a reagent immobilized on the coating layer c) capable of detecting an analyte.

The reagent of the invention comprises a core of a particle functionalized for binding of element b).

"In order" means from inside to outside of the reagent.

"Particle", as used herein, is used for designating colloidal systems of sphere, rod, polyhedron shapes, etc., or similar shapes, individually or forming organized structures (dimers, trimers, tetramers, etc.).

In a preferred embodiment the particle a) has a size above 0.3 µm, preferably 0.3 to 8 µm, more preferably 0.3 to 7 µm, more preferably 0.3 to 6.5 µm, more preferably 0.3 to 6 µm, more preferably 0.3 to 5.5 µm, more preferably 0.3 to 5 µm, more preferably 0.3 to 4.5 µm, more preferably 0.3 to 4 µm, more preferably 0.3 to 3.5 µm, more preferably 0.3 to 3 µm, more preferably 0.3 to 2.5 µm, more preferably 0.3 to 2 µm, more preferably 0.3 to 1.5 µm, more preferably 0.3 to 1 µm. In another preferred embodiment the particle a) has a size of 0.3-0.8 µm. In another preferred embodiment the particle a) has a size of 2-3 µm. In another preferred embodiment the particle a) has a size of 3-4 µm. In another preferred embodiment the particle a) has a size of 4-5 µm. In another preferred embodiment the particle a) has a size of 5-6 µm. In another preferred embodiment the particle a) has a size of 6-7 µm. In another preferred embodiment the particle a) has a size of 7-8 µm. In another preferred embodiment the particle a) has a size of 0.3-0.8 µm. In another preferred embodiment, the particle a) has a size of 0.45 µm. In another preferred embodiment the particle a) has a size of 0.5-1 µm. In another preferably embodiment, the particle has a size of 2.5-4.5 µm or 0.5-1 µm; preferably has a size of 2.8-4.5 µm or 0.5-1 µm. In another preferred embodiment, the particle a) has a size of 1 µm. In another preferred embodiment, the particle a) has a size of 0.5 µm. In another preferred embodiment, the particle a) has a size of 2.7 µm. In another preferred embodiment, the particle a) has a size of 2.8 µm. In another preferred embodiment, the particle a) has a size of 4.5 µm.

The term "particle size" as used herein means the diameter of the particle if the particle is spherical or, if the particle is non-spherical, the volume-based particle size. The volume- based particle size is the diameter of the sphere that has the same volume as the non-spherical particle in question. The values of particle size obtained by the techniques known in the art are in agreement within the experimental error.

The size of the particle can be determined by any method known in the art. As a way of illustrative non-imitative examples, the size of the particle can be determined by sieves, sedimentation, electrozone testing, laser diffraction, flow cytometer, microscopy such as scanning or transmission electron microscopy (SEM or TEM), spectroscopic techniques such as Dynamic Light Scattering or UV-vis spectroscopy, or Eyecon2^{™} . In a preferred embodiment, the size of the particle a) according to the invention correspond to the size determined by Transmission Electron microscopy (TEM), through the images acquired based on a 2D projection of 3D particles, and analyzed by specialized software which determine the particle size distribution from the counting of a relatively large number of particles and their statistical analysis. Other techniques such as flow cytometer can be also employed to estimate the medium size, by correlating their light scattering properties (forward scatter (FSC) and side scatter (SSC)).

In a preferred embodiment, the size of the particle a) refers to a mean size. The mean particle size of various types of particles may differ from the particle size distribution of the particles. For example, a particle can have a mean particle size of about 0.5 µm while its particle size distribution can generally vary between about 0.3 µm to about 0.8 µm. (It should be understood that the particle sizes that are described herein are for particles when they are dispersed in an aqueous medium and the mean particle size is based on the mean of the particle number distribution).

The functionalization of particle a) may be done by covalent or electrostatic (layer-by-layer) binding, by means of an amino, carboxylic acid, epoxy, sulphonate, phosphate, thiol, chlorosulfonyl, alkene, alkyne and azide functional group, more preferably an amino, carboxylic acid or epoxy functional groups. In a preferred embodiment, the electrostatic coating is performed using polycations and polyanions such as Polyethyleneimine (PEI), Poly(ethylene glycol) (PEG) or poly(sodium styrene 4-sulfonate) (PSS), polydiallyldimethylammonium chloride (polyDADMAC), poly(allylamine)-Nafion / poly (acrylic acid)(PAH-Naf / PAH-PAA), linear N, N-dodecyl,methyl-poly(ethyleneimine) / poly (acrylic acid) (DMLPEI/PAA), poly (allylamine hydrochloride) (PAH), poly (allylamine / poly(acrylic acid) (PAH-PAA), poly (acrylic acid) / polyethylene oxide - block - polycaprolactone (PAA/PEO-b-PCL), . In a preferred embodiment, the particle a) has a stable electrostatic coating. In a more preferred embodiment the particle a) is functionalized with an epoxy functional group. In another preferred embodiment, the particle a) is functionalized with an amino functional group. In another preferred embodiment, the particle a) is functionalized with a carboxylic functional group.

In another preferred embodiment, the functionalization of particle a) is done by covalent binding. In another preferred embodiment, the functionalization of particle a) is done by electrostatic binding, more particularly by the technique layer by layer.

Layer-by-layer or (LbL) deposition is a thin film fabrication technique, wherein the films are formed by depositing alternating layers of oppositely charged materials, normally polycations and polyanions (charged polymers), with wash steps in between. Any method known in the art can be used, for example the methods disclosed in ACS Nano 2019, 13, 6, 6151-6169.

The particle a) may be a silica, titania, polystyrene, melamine, latex or other rubber microspheres or polystyrene particle. In a preferred embodiment, the particle a) is a polystyrene particle. In another preferred embodiment, the particle a) is a magnetic polystyrene particle.

In another preferred embodiment, the particle a) has superparamagnetic properties.

"Superparamagnetism" is a form of magnetism exhibited by small ferromagnetic or ferrimagnetic nanoparticles. At sizes of less than a hundred nanometers, the nanoparticles are single-domain particles, allowing the magnetization of the nanoparticles to be approximated as one giant magnetic moment by summing the individual magnetic moments of each constituent atom.

In a more preferred embodiment, the particle used is a uniform microsphere with superparamagnetism properties (MBs), 1 µm in diameter, with an epoxy functional group.

Element b) of the reagent of the invention comprises a shell of a plurality of nanoparticles of a plasmonic material immobilized on the surface of the particle a) wherein the surface of the nanoparticles is coated with aliphatic, aromatic and/or polymeric molecules and at least one Raman label. In a preferred embodiment, the surface of the nanoparticle is coated with any combination of aliphatic, aromatic and/or polymeric molecules and at least one Raman label.

In another preferred embodiment the surface of the nanoparticles is coated with at least two molecules selected from the group consisting of aliphatic, aromatic and/or polymeric molecules. By illustrative non-limitative example the surface is coated with an aliphatic and an aromatic molecule; with two aromatic molecules, or with an aromatic and a polymeric molecule.

Thus, the plurality of nanoparticles immobilized on the surface of particle a) are forming a cluster.

These nanoparticles need to be stable and having an intense Raman signal. This can be achieved by for example
1) the use of two molecules:
   a) a molecule that has Raman properties,
   b) and another molecule that acts as a stabilizer, such as for example aliphatic, aromatic and/or polymeric molecules.
2) the use of a single molecule that has Raman properties and that at the same time stabilizes the nanoparticle.

"Stable" nanoparticle as used herein relates to stability in terms of aggregation and surface chemistry, so the dimensionality and functionality are preserved during the storage or experiment.

Molecules having Raman properties and capable of stabilizing the nanoparticles are widely known in the art. Illustrative non limitative examples of molecules with Raman properties that usually provide stability to nanoparticles usually have charged functional groups (COOH, NH2, etc.), mostly negative, such as a carboxylic acid (COOH). Illustrative non limitative examples of Raman labels are 4-mercaptobenzoic acid (4-MBA), 3-mercaptobenzoic acid (3-MBA), 4-aminothiophenol (4-ATP) or 2-Mercapto-4-methyl-5-thiazole acetic acid.

"Plurality", as used herein, relates to the amount of nanoparticles that allow complete coating of the surface of the particle a). By "complete", as used herein, relates to at least 50% of the surface of the particle a) covered by the nanoparticles, more preferably at least 60%, more preferably at least 65%, more preferably at least 70%, more preferably at least 75%, more preferably at least 80%, more preferably at least 85%, more preferably at least 90%, more preferably at least 95%. As illustrative non limitative example, around 300 nanoparticles are coating a core particle of 1 µm.

"Nanoparticle" is used herein for designating colloidal systems of sphere, rod, polyhedron shapes, etc., or similar shapes, having a size less than 1 micrometer (µm), individually or forming organized structures (dimers, trimers, tetramers, etc.), dispersed in a fluid (aqueous solution). In a particular embodiment, the nanoparticles suitable for putting the invention into practice have a size less than 1 µm, generally comprised between 1 and 999 nanometers (nm), typically between 5 and 500 nm, preferably between 10 and 150 nm, approximately. The shape of the nanoparticles can vary greatly; advantageously, said nanoparticles will adopt any optically efficient shape such as spheres, rods, stars, cubes, polyhedra or any another variant as well as complex associations of several particles; in a particular embodiment, the shape of the nanoparticles for putting the invention into practice is spherical, or it has the shape of a rod, cube, octahedron, decahedron, star, etc. Methods of preparing metallic nanoparticles are well-known to those of skill in the art (Lee, Meisel J. Phys. Chem., (1982) 86, 3391-3395; Baker et al. Anal. Bioanal. Chem., (2005) 382, 1751-1770.2005), and are not further described herein. The nanoparticles of plasmonic materials, e. g., metallic nanoparticles, may be of a suitable size and type.

The nanoparticles suitable for putting the method of the invention into practice can be obtained by conventional methods known by the persons skilled in the art. By way of non-limiting illustration, the production of the nanoparticles for putting the invention into practice is carried out by means of synthetic methods ranging from "top-down" to "bottom-up" type methods including chemical methods comprising, for example, metal salt reduction, overgrowth, light ablation, or physical methods, for example, physical or chemical vapor evaporation, sputtering, milling, etc. The plasmonic particles are encoded with molecules with Raman activity or which can develop Raman activity including organic systems, inorganic systems, organometallic systems, etc., with or without functionalization. The Raman code can be added before, after, or at the time the coating with aliphatic, aromatic and/or polymeric molecules. The ligand can be stabilized on the surface of the nanoparticles by means of suitable chemical techniques, for example, depending on the functional groups eventually present on the surface of the nanoparticles and on the ligand, such as, for example, creation of bonds by means of chemical synthesis or self-assembly, or by means of physical phenomena based on electrostatic or hydrophobic interactions.

In a preferred embodiment, the nanoparticles are synthesized by the method disclosed in Mir-Simon, B., et al. Chem. Mater 27(3), 950-958 (2015) and EP3227037A1 and in the examples of the present invention.

In a preferred embodiment, the size of the nanoparticles is between 5 to 200 nm, preferably 10 to 180 nm, more preferably 15 to 175 nm, more preferably 20 to 170 nm, more preferably 25 to 165 nm, more preferably 30 to 160 nm, more preferably 35 to 155 nm, more preferably 40 to 150 nm, more preferably 45 to 145 nm, more preferably 50 to 140 nm, more preferably 55 to 135 nm, more preferably 60 to 130 nm, more preferably 65 to 125 nm, more preferably 70 to 120 nm, more preferably 75 to 115 nm, more preferably 80 to 110 nm, more preferably 85 to 100 nm. In another preferred embodiment, the size of the nanoparticles is between 30-90 nm, more preferably the size of the nanoparticles is between 60-70 nm. In a more preferred embodiment, the size of the nanoparticles is between 60-75 nm. In a more preferred embodiment, the size of the nanoparticles is 70 nm. In another more preferred embodiment, the size of the nanoparticles is 60 nm. In another more preferred embodiment, the size of the nanoparticles is 65 nm. In another preferred embodiment, the size of the nanoparticles I between 30-55 nm, more preferably 30 to 50 nm, more preferably 30-45 nm, more preferably 30-35 nm, more preferably 35-40 nm, more preferably 40-45 nm, more preferably 40-55 nm, more preferably 45-55 nm, more preferably 45-50 nm or more preferably 50-55 nm.

In a preferred embodiment, the size of the nanoparticle according to the invention corresponds to the size determined by microscopy TEM or UV-vis spectroscopy.

In a preferred embodiment, the particle size refers to the mean particle size.

The definitions and embodiments in relation to particle size are accordingly applicable to the nanoparticle size.

According to the invention, the nanoparticles of element b) are nanoparticles of a plasmonic material.

"Plasmonic material", as used herein, relates to those kind of materials that can sustain surface plasmon wave on metal dielectric interface. Almost any metal-dielectric interface can sustain surface plasmon waves if excited by a light source of suitable frequency via different exciting mechanisms such as prism excitation. The plasmonic materials provide the electromagnetic field necessary for enhancing the SERS signal of the Raman molecule.

In a preferred embodiment, the plasmonic material is selected from the group consisting of gold, silver, copper, palladium, rhodium, aluminium, ruthenium, indium, alkaline, alkaline-earth metals, metals alloys thereof, CuSe, CuTe, silicon and the combinations thereof, particularly silver or gold. In a particular embodiment, the plasmonic material comprises, but is not limited to gold, silver, or copper, aluminium, or alloys thereof, or a combination thereof. In a specific, non-limiting example, the plasmonic material is gold, silver or copper. In a more preferred embodiment, the plasmonic material is silver.

In a preferred embodiment, the nanoparticle comprises a compound that has Raman properties and that at the same time stabilizes the nanoparticle. In another preferred embodiment the nanoparticle has two molecules, one that acts as a stabilizer and another that acts as a Raman label.

Thus, in a preferred embodiment, element b) of the reagent of the invention comprises at least one Raman label. "At least one Raman label", as used in the present invention refers to at least one type of Raman label. In a preferred embodiment, the element b) comprises one Raman label, preferably two Raman label, more preferably three Raman labels.

"Raman label", or Raman code or Raman probe or Raman reporter as used herein, relates to a molecule responsible of a unique vibrational fingerprint, which enhances its signal when it is in close contact with a plasmonic material, such as a metallic nanoparticle (encoded particle), providing high SERS signals.).

In a preferred embodiment, the at least one Raman label is selected from the group consisting of organic dyes, organometallic compounds and aromatic compounds.

"Organic dye", as used herein, relates to an organic compound capable of reflecting certain wavelengths depending on the different chromophores groups present in its molecular structure that are the groups responsible for this property.

"Organometallic compound", as used herein, relates to a chemical compound which contains at least one bond between a metallic element and a carbon atom belonging to an organic molecule.

"Aromatic compound", also known as "mono- and polycyclic aromatic hydrocarbons", as used herein, are organic compounds containing one or more aromatic rings.

In a more preferred embodiment, the organic dye is selected from the group consisting of rhodamines, indole derivatives, azo dyes, porphyrins, perylenes, acridines, anthraquinones, arylmethanes, indamines, oxazones, oxazines, indophenols, thiazines, xanthens and fluorenes.

In another more preferred embodiment, the organometallic compound is selected from the group consisting of porphyrins, phthalocyanines and organic lacquers coordinated with transition metals.

In another more preferred embodiment, the aromatic compound is selected from the group consisting of thiolated, aminated and nitrated derivatives of the benzene, naphthalene, pyrene, anthracen, particularly benzenethiol or naphthalenethiol.

Preferably, the Raman label is selected from the group consisting of 2-mercaptopyridine; benzenethiol; 4- mercaptobenzoic acid; 4-nitrobenzenethiol; 3,4-dicholorobenzenethiol, 3-fluorothiophenol; 4-fluorothiophenol; 3-5-bis(trifluoromethyl)benzenethiol; methylene blue; nile blue A; rhodamine 6G; Toluidine Blue O, 2-Phenylethanethiol, 4-Mercaptophenol, Biphenyl-4-thiol, 7-Mercapto-4-methylcoumarin, 4-(Hydroxyphenyl)-1H-tetrazole-5-thiol, 2-Fluorothiophenol, Crystal Violet, 2-Naphthalenethiol, 4-(((3-Mercapto-5-(2-methoxyphenyl)-4H-1,2,4-triazol-4-yl)imino)methyl)phenol, (2-Trifluoromethyl)benzenethiol, 4-Aminothiophenol, 1-Naphthalenethiol, 1,1',4,1"-Terphenyl-4-Thiol, Biphenyl-4,4'-dithiol, Thiosalicylic acid, 4-(((3-Mercapto-5-(2-pyridinyl)-4H-1,2,4-triazol-4-yl)imino)methyl)-1,2-benzenediol, 4-(((3-Mercapto-5-(2-pyridinyl)-4H-1,2,4-triazol-4-yl)imino) methyl)benzoic, 2,3,4,6-Tetrafluorobenzenethiol, and (5-(4-Methoxyphenyl)-1,3,4-oxidazole-2-thiol).

According to the invention, the surface of the nanoparticles is coated with aliphatic, aromatic and/or polymeric molecules.

In a preferred embodiment, the aliphatic, aromatic and/or polymeric molecule is selected from the group consisting of organic dyes, organometallic compounds, aromatic compounds, polymeric compounds such as poly(ethylene glycol) (PEG), polyethyleneimine (PEI), poly(sodium styrene sulfonate), 1-ethenylpyrrolidin-2-one (PVP), polyelectrolytes or detergents such as cetyltrimethylammonium bromide (CTAB) and anionic poly(sodium 4-styrenesulfonate) (PSS), thiolated molecules such as mercaptoundecanoic acid or mercaptopropanoic acid, 6-mercaptohexanoic acid, 4-mercaptobutyric acid, 8-mercaptooctanoic acid, 12-mercaptododecanoic acid, 16-mercaptohexadecanoic acid, catechol compounds such as 3,4-Dihydroxyhydrocinnamic acid, 3,4-dihydroxyphenylacetic acid, caffeic acid, dopamine, 3,4-Dihydroxy-L-phenylalanine, DL-Norepinephrine hydrochloride and combinations thereof.

The reagent of the invention comprises at least one layer of a coating material covering element b) that interacts with element d). In a preferred embodiment, the reagent comprises one layer, preferably two layers, more preferably three layers of the coating material.

This external coating can: i) prevent the code from leaching out into the medium thus avoiding toxic effects or vibrational cross contamination with the codes of other particles; ii) protect the plasmonic particle from contaminations of the medium that may give rise to vibrational noise hindering the particle readout; iii) increase the colloidal stability of the particle; iv) provide a convenient surface for further chemical functionalization; and, v) protect the plasmonic core for interacting with other plasmonic particles avoiding plasmon coupling and so the uncontrolled generation of hotspots.

The coating material used according to the invention must be transparent for Raman signal and/or compatible with Raman signal that is, the imaginary component of the refractive index of the material is zero. The transparency of a material can be determined by Raman spectroscopy.

The chemical nature of said coating can vary greatly; by illustrative, the coating may be formed of any suitable material known in the art; for example, and not wishing to be limiting in any manner, it may comprise silica, or a suitable metal oxide, or one or more than one polymer, or block copolymer, etc. In a preferred embodiment, the coating material is selected from the group consisting of a metal oxide, a polymer, a protein, a polysaccharide and a lipid.

In a more preferred embodiment, the metal oxide is selected from the group consisting of silicon dioxide, titanium dioxide, aluminium oxide, zinc oxide, cerium oxide and zirconium oxide.

In another preferred embodiment, the polymer is selected from the group consisting of polyethylene glycol (PEG), sulfonated polystyrene (SPS), polystyrene (PS), styrene, acrylate derivatives, maleimide, polyether ether ketone (PEEK), polyvinylpyrrolidone (PVP), epoxy resins, diglycidyl ether of bisphenol A (DGEBA) and poly(butyl acrylate).

In a preferred embodiment, the coating material is silicon dioxide, also known as silica, or titanium dioxide, also known as titanium(IV) oxide or titania. In another preferred embodiment, the coating material is silica. In another preferred embodiment, the coating material is titania.

The coating, in general, and silica or titania, in particular, provides the core (which is surrounded/ coated by the coating) with mechanical and chemical stability, prevents the core from exterior reactions, and renders the core amenable to use in many solvents without disrupting the SERS response. Further, the coating prevents other analytes from entering SERS hot sites to displace the signal of the Raman active reporter molecules. Additionally, the coating enables attachment of suitable ligands (e.g., biomolecules). This core and coating structure is well known for the skilled person in the art. Methods for preparing a coating comprising silica or titania are also well-known to those of skill in the art.

Silica coating can be performed for example by a method disclosed in Stöber, W., et al. J. Colloid Interface Sci. 26, 62-69 (1968). Thus, a precursor such as tetraethyl orthosilicate alkoxide (TEOS) is added under basic conditions to achieve its hydrolysis and condensation. This process must be well controlled to avoid nucleation of silica particles, and ensure the reagent is only placed on the surface of the magnetic cluster. One, two, three or more layers of silica can be synthesized to increase silica stability in aqueous solutions.

As mentioned above, cluster coating can be also performed with titania, which usually has greater stability in aqueous media. Thus, in another preferred embodiment the coating material is titanium dioxide. This coating can be carried out through the deposition of small TiO₂ nanoparticles on the surface of silver nanoparticles (Yang, X.H., et al. Nanotechnol. 24, 415601(2013)). Titanium precursors such as titanium (IV) isoproxide (TTIP) and ethylene glycol in acetone can be used, obtaining titanium glycolate prior to the formation of titania nanoparticles when the complex is heated. In this case, phosphoamines such as 2-aminoethyl dihydrogen phosphate can be used.

In another more preferred embodiment, the saturated fatty acid is selected from the group consisting of lauric acid, myristic acid, palmitic acid, and stearic acid. In another preferred embodiment, the unsaturated fatty acid is selected from the group consisting of palmitoleic acid, oleic acid, linoleic acid, and linolenic acid.

In another more preferred embodiment, the protein is selected from the group consisting of bovine serum albumin and casein.

In another more preferred embodiment, the polysaccharide is selected from the group consisting of cellulose, dextran, glycogen, chitin, galactogen and starch.

In another embodiment, the lipid is an aliphatic lipid.

"Aliphatic lipid", as used herein, relates to lipids that contain carbon chains which may be straight and saturated or branched or unsaturated, in one or more positions.

The thickness of the coating may vary. By illustrative, and without wishing to be limiting in any manner, the thickness of the coating may be applied in a controlled manner over the core Raman active reporter molecules and the aliphatic, aromatic and/or polymeric molecule or combinations thereof. The thickness of the coating, once complete, may be about 1 nm and 400 nm, or any value there between; for example, the silica or titania coating may be about 1, 10, 20, 25, 30, 35, 40, 45, 50, 55, 60, 65, 70, 75, 80, 85, 90, 95,100, 150, 200, 250, 300, 350 or 400 nm thick, or any value there between. In a preferred embodiment, the layer c) of coating material has a thickness of 1 to 300 nm; preferably from 1 to 120 nm. Several methods known in the art can be used to determine the thickness of the coating. In a preferred embodiment, the thickness of the coating is determined by transmission electron microscopy (TEM).

Element d) of the reagent of the invention comprises a reagent immobilized on the coating layer c) capable of detecting an analyte.

"Reagent capable of detecting an analyte", or ligand, as used herein, refers to a compound which is used for probing the presence of the target analyte. In its broadest sense, the term includes any substance with the capacity to bind to a target analyte, and particularly any molecule capable of giving rise to selective interactions with the target analyte. The person skilled in the art will understand that the chemical nature of the ligand will depend on the chemical nature of the target analyte. Preferably selective ligands, for a wide variety of target analytes, are known or can be easily found using known techniques. By way of non-limiting illustration:
- when the target analyte is a protein, the ligand can be a protein (for example, an antibody or a fragment thereof (Fabs, etc.));
- when the target analyte is an enzyme, the ligand can be a substrate or an inhibitor of said enzyme;
- when the target analyte is a nucleic acid-binding protein or a nucleic acid, the ligand can be an aptamer, such as a peptide aptamer or a DNA or RNA aptamer; and
- when the target analyte is a nucleic acid, the ligand can be a nucleic acid, such as a probe, etc.
- Therefore, in a particular embodiment, the ligand is an antibody, or a fragment thereof, recognizing the target analyte.

Advantageously, the reagent capable of recognizing the analyte is a molecule giving rise to a selective interaction with the target analyte; i.e., the ligand has the capacity to distinguish between several target analytes present in the sample. Illustrative examples of such selective interaction include the antigen-antibody interactions, enzyme-substrate interactions, key-lock interactions, etc.

In a preferred embodiment, the reagent capable of detecting an analyte is selected from the group consisting of an antibody or functional fragments thereof, aptamers, enzymes, nucleic acids, and peptides. In a more preferred embodiment, the reagent capable of detecting an analyte is an antibody.

As used herein, the term "antibody", or "immunoglobulin" (Ig) refers to gamma-globulin type glycoproteins. The typical antibody is made up of basic structural units, each of which with two large heavy chains and two smaller light chains, forming monomers (with one unit), dimers (with two units) or pentamers (with five units), for example. In this context, the term "antibody" includes natural antibodies, monoclonal antibodies, polyclonal antibodies, recombinant antibodies, etc. Likewise, the term "antibody" refers both to complete antibodies and to fragments thereof maintaining the capacity to bind to the corresponding antigen, as well as to recombinant antibodies, such as for example, single-chain Fv fragments (scFv), diabodies, single-domain antibodies (VH), nanobodies (VHH), etc.

In a preferred embodiment, the coating layer further comprises a compound that allows the immobilization of the reagent capable of detecting an analyte and/or the coating material is modified to interact with element d).

In case that the layer of the coating material further comprises a compound that allows the immobilization of the reagent capable of detecting an analyte and said reagent is an antibody, the compound that allows its immobilization can be selected from the group consisting of silanes, phosphines, thiols, carboxylic acids, amines, epoxides, and catechols.

In a preferred embodiment, the silane is selected from the group consisting of (Aminopropyl)trimethoxysilane (APTMS), (3-Aminopropyl)triethoxysilane, 3-Glycidyloxypropyl trimethoxysilane (GPTMS), (3-Glycidyloxypropyl)triethoxysilane, (3-Mercaptopropyl)trimethoxysilane, (3-Mercaptopropyl)triethoxysilane, and 3-Mercaptopropyl trimethoxysilane (MPTMS).

In another preferred embodiment, the phosphine is selected from the group consisting of 2-aminoethyl dihydrogen phosphate, 4-Aminobutylphosphonic acid, 3-Aminopropylphosphonic acid, (Aminomethyl)phosphonic acid, 4-Aminobenzylphosphonic acid, 6-Phosphonohexanoic acid, 3-Phosphonopropionic acid, Phosphonoacetic acid, 11-Hydroxyundecylphosphonic acid and O-Phosphorylethanolamine-1,2-13C2.

In another preferred embodiment, the thiol is selected from the group consisting of mercaptoundecanoic acid or mercaptopropanoic acid, 6-mercaptohexanoic acid, 4-mercaptobutyric acid, 8-mercaptooctanoic acid, 12-mercaptododecanoic acid, 16-mercaptohexadecanoic acid, 6-Amino-1-hexanethiol hydrochloride, 8-Amino-1-octanethiol hydrochloride, 3-Amino-1-propanethiol hydrochloride, 11-Amino-1-undecanethiol hydrochloride, 16-Amino-1-hexadecanethiol hydrochloride, and Cysteamine.

In another preferred embodiment, the catechol is selected from the group consisting of 3,4-Dihydroxyhydrocinnamic acid, 3,4-dihydroxyphenylacetic acid, caffeic acid, dopamine, 3,4-Dihydroxyphenethylamine hydrochloride, 3,4-Dihydroxy-L-phenylalanine, Dopamine hydrochloride and DL-Norepinephrine hydrochloride.

The reagent capable of detecting an analyte (for example antibodies) is immobilized on the coating layer c). This can be done through electrostatic adsorption and covalent binding by standard protocols (Dynabeads^{™}, BangsLab Protocols, etc.). Covalent immobilization is normally preferred. In a preferred embodiment, the well-known carbodiimide reaction can be employed to obtain peptide bonds when carboxylic acid or amine functional groups of the coated material from the cluster are used (Sci. Rep. 6, 29014 (2016); Adv. Mater. Technol. 1 (8), 1600163 (2016)). In another preferred embodiment, epoxy groups (such us for GPTMS) can be also employed to directly attach the reagent capable of detecting an analyte to the coated material of the cluster. In another preferred embodiment, intermediate proteins can be used to bind the reagent capable of detecting an analyte to the coated cluster, such as the case of protein A/G or the complex biotin/streptavidin. In another preferred embodiment, in case of antibodies or their parts, thiol groups can be also employed to bind them to the coated surface of the reagent. In all cases, standard protocols can be employed.

To minimize non-specific adsorptions during the biochemical assay, such as cell debris or blood proteins, final surface can be blocked with buffer solutions containing proteins and/or surfactants, such as BSA and/or Tween 20^{®}, casein, Tris buffer, among others.

Flow cytometry analysis can confirm the correct binding. For instance, in case of antibody binding, a secondary antibody labelled with a fluorophore is added to the reagent. In a correct binding of the antibody, the reagent will show fluorescence due to the binding of the secondary antibody to the primary one. On the contrary, no fluorescence will be observed in case of failed binding of the primary antibody. An example of biofunctionalized beads has been also provided.

"Analyte", as used herein, relates to a chemical compound, a bacterium, yeast, fungus, virus, mammalian cell, parasite, viroid, or toxin to be identified or measured.

In a preferred embodiment, the analyte is one of eukaryotic and prokaryotic cells, including circulating tumoral cells, pathogenic and non-pathogenic microorganisms such as fungi, protozoa, algae, rotifers, bacteria and archaea, viruses, nucleic acids, peptide nucleic acids, antigens, peptides and proteins or the combination thereof. In a more preferred embodiment, the analyte is a pathogenic microorganism. In another preferred embodiment, the analyte is a circulating tumor cell.

"Circulating tumor cell", as used herein, relates to a cancer cell that splits away from the primary tumor and appears in the circulatory system as singular units or clusters.

"Chemical compound", as used herein, include antibodies, antigens, peptides or nucleotide sequences.

In the sense used in this description, the term "nucleic acid" refers to polymers formed by the repetition of nucleotides bound by means of phosphodiester bonds. This term includes viroids, transposons, retrotransposons and plasmids or vectors.

In the sense used in this description, the term "microorganism" includes very small or microscopic organisms which can be unicellular or multicellular organisms. The concept of microorganism lacks any taxonomic or phylogenetic implication since it encompasses unicellular organisms not related to one another, such as prokaryotes (for example, bacteria (including mycoplasmas) and archaebacteria), eukaryotes (for example, protozoa, fungi, algae, microscopic plants (green algae) and microscopic animals (for example, rotifera and planarias). Non limiting illustrative examples of said microorganisms include pathogenic bacteria and non-pathogenic bacteria of interest, such as for example, *Bacillus, Vibrio,* for example, *V. cholerae, Escherichia,* for example *E. coli, E. enterotoxigenic,* Shigella, for example, *S. dysenteriae, Klebsiella,* for example, *K. pneumoniae,* , *Enterobacter,* for example, *E. cloacae, Salmonella,* for example, *S. typhi, Mycobacterium* for example *M. tuberculosis, M. leprae, Clostridium,* for example, *C. botulinum, C. tetani, C. difficile, C. perfringens; Corynebacterium,* for example, *C. diphtheriae; Streptococcus,* for example, *S. pyogenes, S. pneumoniae, Staphylococcus,* for example, *S. aureus, S. epidermidis, Enterococcus* , for example, *E. faecalis, E. faecium, Haemophilus,* for example *H. influenzae, Neisseria,* for example, *N. meningitidis, N.gonorrhoeae*; *Yersinia,* for example, *Y. lamblia, Y. pestis, Pseudomonas,* for example, *P. aeruginosa, P. putida; Chlamydia,* for example, *C. trachomatis; Bordetella,* for example, *B. pertussis, Treponema,* for example, *T. pallidum,* etc. In a particular embodiment, said cell is a eukaryotic cell. In a preferred embodiment, the analyte is a bacteria. In another preferred embodiment, the analyte is a yeast. As used herein, yeast includes not only yeast in a strict taxonomic sense, i.e., unicellular organisms, but also yeast-like multicellular fungi of filamentous fungi. In a preferred embodiment, the fungi is *Candida (albicans, krusei, glabrata, tropicalis, parapsilosis, auris, haemoulonii, dublinensis,* etc.), *Cryptococcus neoformans, Aspergillus (fumigatus, niger,* etc.), *Genus Mucorales (mucor, absidia, rhizophus), Sporothrix schenkii, Blastomyces dermatitidis, Paracoccidioides brasiliensis, Coccidioides immitis* or *Histoplasma capsulatum.* In another preferred embodiment, the parasite is *Entamoeba histolytica, Balantidium coli, Naegleria fowleri, Acanthamoeba sp., Giardia lamblia, Cryptosporidium sp., Pneumocystis carinii, Plasmodium vivax, Babesia microti, Trypanosoma brucei, Trypanosoma cruzi, Leishmania donovani, Toxoplasma gondii* or *Nippostrongylus brasiliensis.*

In the sense used in this description, the term "virus" includes infectious agents having a small size and simple composition which can only multiply in animal, plant or bacteria cells in which they live. They are made of a nucleic acid (DNA or RNA) and of a protein layer. Non-limiting illustrative examples of viruses which can be detected by means of the present invention include orthomyxovirus (for example, flu virus, etc.), paramyxovirus (for example, respiratory syncytial virus, mumps virus, measles virus, etc.), adenovirus, rhinovirus, coronavirus (for example SARS-CoV-2), reovirus, togavirus (for example, rubella virus, etc.), parvovirus, smallpox virus, vaccinia virus, enterovirus (for example, poliovirus, etc.), hepatitis virus (including hepatitis A, B, C virus, etc.), herpesvirus (for example, herpes simplex virus, varicella-zoster virus, cytomegalovirus, Epstein-Barr virus, etc.), rotavirus, Norwalk virus, hantavirus, arenavirus, rhabdovirus (for example, rabies virus, etc.), retrovirus (for example, HIV, the HTLV-I and HTLV-II, etc.), papovavirus (for example, papillomavirus, etc.), polyomavirus, picornavirus, etc.

In a preferred embodiment, the analyte is a mammalian cell. In another preferred embodiment, the analyte is a cancer cell, an antigen expressed at the surface of the tumor cell, or an antigen secreted by the tumor cell. Examples of cancer or tumor include without limitation, breast, heart, lung, small, intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytic tumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelial tumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor.

Amongst tumor antigens expressed by tumor cells commonly glycoproteins, peptides, carbohydrates, and glycolipids, are included. Non-limiting examples of tumor associated antigens include: AFP (Alpha (α)-fetoprotein), AIM-2 (Interferon-inducible protein absent in melanoma 2), ART-4 (Adenocarcinoma antigen recognized by T cells 4), BAGE (B antigen), BCMA, CAMEL (CTL-recognized antigen on melanoma), C16a, CD19, CD20, CD22, CD30, CD3, CD40, CD33, CD123, VEGF, IL-6, MUC-1, endoglin, DLL, B7-H3, CEA (Carcinoembryonic antigen), DAM (Differentiation antigen melanoma), Ep-CAM (Epithelial cell adhesion molecule), ErB3, FAP, gpA33, Her2, IGF-1R, CD-5, FAP, MAGE (Melanoma antigen), MART-1/Melan-A (Melanoma antigen recognized by T cells-1 / melanoma antigen A), MC1R (Melanocortin 1 receptor), MET, MUC-1, NY-ESO-1 (New York esophageous 1), OA1 (Ocular albinism type 1 protein), P-Cacherin, PD-L1, PSMA (Prostate-specific membrane antigen), SART-1, -2, -3 (Squamous antigen rejecting tumor 1, 2, 3), Survivin-2B (Intron 2-retaining surviving), TRP (Tyrosinase-related protein).

In another preferred embodiment, the analyte is a biomarker known to be suitable for diagnosing a disease.

In another aspect, the invention relates to a kit comprising the surface-enhanced Raman scattering-(SERS)-active reagent according to the invention.

The term "kit", as used herein, refers to a product containing the different reagents necessary for carrying out the method of detecting, identifying and/or quantifying an analyte in a sample packed so as to allow their transport and storage. Materials suitable for packing the components of the kit include crystal, plastic (e.g. polyethylene, polypropylene, polycarbonate), bottles, vials, paper, or envelopes.

Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media, optical media, or audio materials. Additionally or alternatively, the media can contain internet addresses that provide said instructions.

### Methods of the invention

In another aspect, the invention relates to a method for detecting, identifying and/or quantifying an analyte in a sample, which comprises the use of the SERS active reagent according to the invention.

In another aspect, the invention relates to a method for detecting and identifying the presence and/or the amount of at least one analyte in a sample, the method comprising:
a) incubating the sample comprising the analyte with a reagent capable of recognizing the analyte, during a time sufficient to allow the formation of a complex between the reagent and the analyte;
b) separating the complex of the reagent and the analyte from the sample;
c) optionally, staining the analyte with a fluorescence label;
d) eluting the analyte from the complex
e) incubating the eluted analyte, with at least one Raman tag selective/specific for the analyte during a time sufficient to allow the labelling of the eluted analyte with the selective Raman tag; and
f) acquiring an image of the sample with an optical and/or fluorescence microscope and
g) measuring Raman spectroscopy to detect and identify the presence and/or amount of the at least one analyte.

All the particular embodiments of the reagent of the present invention are also applicable to the method for detecting and identifying the presence and/or the amount of at least one analyte in a sample of the invention.

Step a) of the method of the invention comprises incubating the sample comprising the analyte with a reagent capable of recognizing the analyte, during a time sufficient to allow the formation of a complex between the reagent and the analyte.

To minimize non-specific adsorptions during step a), such as cell debris or proteins, the reagent capable of recognizing the analyte can be blocked with buffer solutions containing proteins and/or surfactants, such as BSA, casein and/or Tween 20^{®}, Brij, SDS, among others.

"Sample", as used herein, relates to biological material isolated from a subject and therefore includes biological samples. Said sample can contain any biological material suitable for detecting the desired analyte and can comprise cells and/or non-cellular material from the subject. In general, a sample can be isolated from any suitable biological tissue or fluid. In a preferred embodiment, the sample is a fluid sample.

As used herein, the term "fluid" encompasses liquids and gases. Non-limiting illustrative examples of fluids include solutions, suspensions, etc., in which the target analyte can be dissolved or dispersed in a suitable medium, such as a medium, or aqueous-organic medium. In preferred embodiment, the fluid sample is selected from the group consisting of blood, serum, plasma, ascites, synovial fluid, peritoneal fluid, pus, amniotic fluid, lymph fluid, pericardial fluid, cervicovaginal fluid, urine, semen, sputum, cerebral spinal fluid, tears, mucus, sweat, milk, brain extracts and saliva. In a more preferred embodiment, the sample is blood.

In a preferred embodiment, the fluid sample is diluted before step a). The sample can be diluted 1:0.1, 1:0.2, 1:0.3, 1:1, 1:1.1, 1:1.2, 1:1.3 or 1:1.5 for example. Preferably the sample is diluted 1:1. Several reagents or solvents can be used for diluting the same, for example PBS or saline solution.

In a preferred embodiment, the compound capable of detecting an analyte is selected from the group consisting of an antibody or functional fragments thereof, aptamers, enzymes, nucleic acids, and peptides. In a more preferred embodiment, the compound capable of detecting an analyte is an antibody. As a way of illustrative non-limitative example, the reagent capable of recognizing the analyte can be any compound which can bound to the analyte, for example diverse type of commercial particles can also be used, such as polystyrene, latex or silica particles or magnetic beads (MBs) surface-modified with selective biomolecules, such as antibodies, aptamers, or peptides. These particles typically have a polystyrene matrix but may also be made of another material such as silica, titania, polystyrene, melamine, latex or other rubber microspheres, or magnetic polystyrene microspheres etc., where nanoparticles with superparamagnetic properties, such as ferrite or magnetite, are embedded. Particles of different sizes can be used, normally 0.5-4.5 µm, preferably 1 to 3.5 µm, more preferably 1 µm or 2.8 µm microspheres. Its surface can be functionalized with a uniform monolayer of molecules containing functional groups such as carboxylic acid, amino, or epoxy, or proteins such as protein A, protein G, streptavidin, avidin and or biotin, which are used to electrostatically or covalently bind biomolecules.

In a preferred embodiment, the reagent in step a) comprises a particle, preferably a magnetic particle. In another preferred embodiment, the particle a) is a polystyrene particle. The use of magnetic particles would not change the procedure, but the cleaning step at the end of the assay. Instead of using the magnetic properties of the particles, centrifugation would be needed. In another preferred embodiment, the reagent in step a) comprises an antibody or fragments thereof, aptamers, enzymes, nucleic acids, or peptides, or any combinations thereof, more particularly an antibody.

In a particular embodiment, the reagent capable of recognizing the analyte is a magnetic particle comprising a covalently bound antibody specific for the analyte.

In this context, "specific" binding is understood as the reagent binding to the target analyte with sufficient specificity so as to distinguish between the target analyte and other components or contaminants of the test sample. The binding must be strong enough for the reagent to remain bound to the target analyte under the test conditions. In a particular embodiment, for example, in the detection of certain biological molecules, the diassociation constant of the target analyte and the reagent will be equal to or less than about 10⁻⁴ M⁻¹, preferably equal to or less than 10⁻⁶ M⁻¹, more preferably equal to or less than 10⁻⁸ M⁻¹, still more preferably equal to or less than 10⁻¹⁰ M⁻¹.

In the assay, a given volume of these concentrated microparticles is mixed with the patient's blood sample. In a particular embodiment, time sufficient to allow the formation of a complex between the reagent and the analyte, incubation time, is from 1 minute to 3 hours, preferably 30 min, more preferably 60 minutes, more preferably 90 minutes, more preferably 12 minutes. The incubation can be performed at several temperatures, for example from 0 to 45 °C. In a preferred embodiment, the incubation is performed at 4 °C. In another preferred embodiment, the incubation is performed at 30 °C. In another preferred embodiment, the incubation is performed at 37 °C.

In a more preferably embodiment, the time is 1 hour of incubation and the temperature is 37 °C. In another preferred embodiment, the incubation time is 1 hour and the temperature is 4 °C.

The separation of the complex of the reagent and the analyte from the sample can be performed by any method, for example separation may be performed by magnetic separation, gravity or by suction, centrifugation, filtration, decantation, or the like or combinations thereof. In a preferred embodiment, the separation is performed by magnetic separation. In another preferred embodiment, the separation is performed by centrifugation. In the case that the reagent in step a) comprises a magnetic particle, the magnetic properties of the particles make possible to concentrate the analyte into a reduced volume, simplifying at the time the washing steps. Selective capture of analytes of the sample is possible through the biorecognition element immobilized on the surface of the complex. In case that the particle a) has no magnetic properties, any known method can be used to separate the analyte from the sample.

The method for detecting the presence and/or the amount of at least one analyte in a sample optionally comprises staining the analyte with a fluorescence label after the separation of step b). In another preferred embodiment, the fluorescent labelling of the analyte can be carried out at the end of step e). In another preferred embodiment, the fluorescent labelling can be performed together with the incubation of the at least one SERS reagent, as long as the fluorophore used does not present adsorption on the SERS Tags.

"Fluorescent label", as used herein, relates to a molecule that is attached chemically to aid in the detection of a biomolecule such as a protein, antibody, or amino acid. Generally, fluorescent tagging, or labelling, uses a reactive derivative of a fluorescent molecule known as a fluorophore. The fluorophore selectively binds to a specific region or functional group on the target molecule and can be attached chemically or biologically.

The fluorescence label is intended for the analyte staining for being detected by fluorescence. To do so, a fluorescent dye or fluorophore can be used. Different options exist depending on the excitation and emission wavelength employed. An example of a label includes, but is not limited to, Blue (488 nm); Green (usually labeled FL1): FITC1 Alexa Fluor 488, GFP, CFSE, CFDA-SE, DyLight 488; Orange (usually FL2): PE, PI; Red channel (usually FL3): PerCP, PerCP- Cy5.5, PE-Alexa Fluor 700, PE-Cy5 (TRICOLOR), PE-Cy5.5 TRITC; Infra-red (usually FL4; not provided by all FACS machines as standard): PE-Alexa Fluor 750, PE-Cy7; Red diode laser (635 nm); APC; APC-Cy7, APC-eFluor 780; CF770; Alexa Fluor 700; Cy5; Draq-5; Violet laser (405 nm); Pacific Orange; Amine Aqua; Pacific Blue; DAPI; Hoescht, Calcofluor White; Alexa Fluor 405; eFluor 450; eFluor 605 Nanocrystals; eFluor 625 Nanocrystals and eFluor 650 Nanocrystals. In addition, when the analyte to be detected is a microorganism, compounds that selectively recognize specific regions of a microorganism (cell surface, DNA, live and dead cells, intracellular structures), which can be or not modified with a fluorophore, could also be used, as Propidium Iodide; Ethidium Homodimer I (EthD-I); Ethidium Homodimer III (EthD-III); DAPI; Hoescht Fluor, Calcofluor White; RedDot^{™}; Live-or-Dye^{™} Fixable Viability Stains; BactoView Red 590; BactoView Live Red; Thiazole orange; Far Red Nuclear Stain; Syto^{®} (Syto9); MitoView^{™};CellBrite^{™} Fix Membrane Stains; BacLight Red 644; Concanavalin-A-AlexaFluor or Concanavalin-A-CF^{®} conjugates for labelling fungi, or Wheat Germ Agglutinin-Alexa Fluor, Wheat Germ Agglutinin-CF^{®} conjugates; Wheat Germ Agglutinin-TRITC for Gram-positive bacteria staining. The use of a nucleic acid marker is preferred in this case, since it allows to stain all type of microorganisms. SYTO^{™} 83 Orange Fluorescent Nucleic Acid Stain has been preferred, which emits in the orange (exc. 543 nm/ ems. 559 nm). The use of compounds that selectively recognize specific regions of microorganisms, such as DAPI (exc. 359 nm/ ems. 457 nm), Hoescht (exc. 352 nm/ ems. 454 nm), Calcofluor White (exc. 350 nm/ ems. 432 nm) are also preferred. In a preferred embodiment, a combination of different fluorescence labels can be used.

This staining allows the detection of microorganisms in an initial screening of the sample, identifying the presence or absence of any bacteria or fungus and estimating the degree of infection of the patient by counting the number of microorganisms. It is worth to mention that fluorophores which are excited and emit fluorescence in the NIR have also been tested. These compounds normally have low stability, losing quickly their fluorescence.

Step d) of the method of the invention for detecting the presence and/or the amount of at least one analyte in a sample comprises eluting the analyte from the complex.

The elution of the analyte from the complex can be performed by several means, for example by using acid or basic buffered solutions which destabilize the complex formed between the analyte and the agent capable of recognizing the analyte, but also by means of high salt concentration solutions, lysing solutions, detergents, or complexing agents, among others.

In a preferred embodiment, the elution is performed using low or high-pH buffers, which dissociates the reagent capable of detecting an analyte, for example the antibody, from the immobilized analyte. Glycine-HCl or citric acid at pH 2-4; or triethylamine, triethanolamine, basic glycine or ammonium hydroxide at pH 10-13, among others, can be employed. In another preferred embodiment, ammonium hydroxide or glycine solution at pH ≥ 10 for at least 5 minutes can be employed. In another preferred embodiment, Glycine-HCl at pH 2, 2.5, 3, 3.5, 4 can be also used.

In another embodiment, solutions with high ionic strength or with of chaotropic effects can be employed, such as high concentration of magnesium chloride, lithium chloride, sodium iodide, or sodium thiocyanate. As example, but not limited to, a solution of KI, MgCl2 or urea at a concentration ≥ 2.5 M.

In another embodiment, protease inhibitors as papain, pepsin, ficin, or trypsin can be also used for antibodies fragmentation, as well as disulphide reducing agents, like dithiothreitol (DTT) or 2-mercaptoethanol.

In another embodiment, immunoprecipitation and/or Western Blot buffers can be also employed, such as RIPA or cell lysis buffers, where detergents are normally included in their composition. In this case, soft conditions are preferred to maintain the analyte structure.

In case of detergents use, non-denaturalizing compounds would be preferred, such as non-ionic detergents. Some examples, but not limited to, would be NP-40, Brij^{™}30, Triton X-100, or Tween20^{®}.

In a more preferred embodiment, ionic detergents can be also used, such as, but not limited to, sodium dodecyl sulphate (SDS) or sodium deoxycholate (SDC). Low concentration of the detergent for a short period of time is preferred, such as 7%, during few seconds, or for 60 minutes if lower concentration than 7% (PLoS One. 2011 Mar 23;6(3):e18218.). These solutions may include the use of some buffer compound, such as TRIS or PBS; the addition of chelating ligands as EDTA or EGTA; some salts like NaCl, NaF, Na₃VO₄; NaN₃; or any other compound as glycerol, etc. Any combination of them can be also possible.

In another embodiment specific competitors may be also employed. In this case, an excess of the counter ligand or an analogue (≥ 0.1 M) should be used to obtain the exchange of the analyte from the microsphere. For example, in case of streptavidin coated microbeads, where the binding is done through an antibody-biotin complex, a solution of biotin or a streptavidin-Binding Peptide (SBP)-Tag can be employed, where other additional compounds can also be included as co-adjuvants. Equivalent strategies can be used in case of microspheres coated with protein A or protein G (EP 2 725 359 B1).

In most cases, conditioning buffers to restore initial conditions are necessary.

In case of basic pH preferred embodiment, an acid pH solution is added once the analyte is isolated from the microspheres until neutral pH is obtained (pH 6-8).

In case of detergents preferred embodiment, dilution of the sample will be necessary until restore physiological conditions.

Step e) of the method of the invention for detecting the presence and/or the amount of at least one analyte in a sample comprises incubating the eluted analyte, with at least one Raman tag selective for the analyte during a time sufficient to allow the labelling of the eluted analyte with the selective Raman tag.

Raman tag selective/specific for the analyte, as used herein, relates to a compound with the capacity to bind to a target analyte and also a Raman active reporter molecule. Said Raman tag selective/specific for the analyte can be composed by two molecules, one having the ability to bind to the target analyte and another as a Raman probe.

In a preferred embodiment, the selective Raman tag is a SERS tag according to the invention.

According to step e), a given volume of SERS Tags is mixed with the eluted (and optionally stained) analyte. In a particular embodiment, time sufficient to allow the formation of a complex between the reagent and the analyte, incubation time, is from 1 minute to 3 hours, preferably 30 min, more preferably 60 minutes, more preferably 90 minutes, more preferably 12 minutes. The incubation can be performed at several temperatures, for example from 0 to 45 °C. In a preferred embodiment, the incubation is performed at 4 °C. In another preferred embodiment, the incubation is performed at 30 °C. In another preferred embodiment, the incubation is performed at 37 °C.

In a more preferably embodiment, the time is 1 hour of incubation and the temperature is 37 °C. In another preferred embodiment, the incubation time is 1 hour and the temperature is 4 °C.

In case of employing magnetic SERS Tags, the analyte can be concentrated and/or cleaned from the dye, if necessary, by magnetic separation.

The step f) of the method of the invention for detecting the presence and/or the amount of at least one analyte in a sample requires acquiring an image of the sample with an optical and/or fluorescence microscope.

According to step f), the sample can be analysed acquiring optical (white light) and/or fluorescence images, and its Raman spectra. A Raman equipment can be used since its usual configuration normally allow white light image acquisition. For fluorescence images acquisition, the Raman microscope should be equipped with a photoluminescence module. Alternatively, white light or fluorescence images and the Raman spectra can be collected separately, with independent and dedicated equipment.

The sample is placed on a solid surface, made preferably of stainless steel, glass, quartz or CaF₂ to minimize Raman background signal. Borosilicate glass supports (glass slides or cover slips), glass fibre or cellulose acetate filters, or thin films of polycarbonate or olefin copolymer can be also employed. The use of inserts or isolators, chamber slides, or perfusion chambers allow to confine the liquid in a specific area. In case of filter measurements, membrane pores can have 0.1 to 25 µm, preferably 1 µm or preferably smaller than 1 µm to retain the analyte on its surface. In case of confined liquid and the use of magnetic SERS Tags, a magnet can help to concentrate the sample in the surface of the material, being feasible even to remove the liquid to measure a dry sample.

As a way of illustrative non-limitative example, an epifluorescence microscope can be used (Nikon Eclipse TS2), which is equipped with a 5 MPixel camera, a CMOS sensor, and a set of ET CY3/TRICT filters. An exposure time from 10 ms until 5 s, for example 500 ms can be used for fluorescence images acquisition.

Commercial image processing programs such as Image J or Particle Analysis (Renishaw plc) can be useful to analyse images. Dedicated codes or algorithms can be also employed. In the analysis of fluorescent images, the absence of any fluorescence would indicate that there are no analytes (pathogens) in the sample, while fluorescent images would indicate the presence of analytes, confirming in case of pathogens be detected thus a blood infection. In this case, the sample should be further scanned for the Raman spectra, which will provide additional information regarding the pathogen identity.

For the Raman spectra acquisition, an excitation laser of 785 nm can be used (RA816 Renishaw plc). In any case, other excitation wavelengths such as 532 nm or 633 nm can also be used.

Step g) of the method of the invention comprises measuring Raman spectroscopy to detect and identify the presence and/or amount of the at least one analyte. Thus, step g) requires analysing the spectra associated with the target analyte-ligand complexes formed, and thereafter identifying the target analyte present in said sample. The analysis of the obtained Raman spectrum can be done by comparing the registered spectra with a Raman spectra library.

Data analysis of SERS spectra can generate colour channel images for each of the analysed RL, obtaining a set of images with different channels. Ideally, only one type of RL should be identified in the fluorescent area scanned. The Raman spectrum detected in the SERS measurement can be correlated with the specific reagent capable of detecting an analyte (for example an antibody) immobilized on the surface of the magnetic SERS Tag, making possible the analyte (for example pathogen) identification in a multiplex analysis.

In a particular embodiment, the method of the invention allows the simultaneous detection (multiplex) of a plurality of target analytes present in a sample, for example, microorganisms (pathogenic or non-pathogenic microorganisms), cells (normal or tumor cells), etc., in a biological or environmental sample.

The person skilled in the art will understand that a large number of target analytes contained in a sample can be detected simultaneously by means of putting the method of the invention into practice; to that end, it is enough to perform step a) by incubating the sample comprising different analytes with a set of different reagents capable of recognizing each analyte, and step e) comprises incubating the eluted analytes with a set of different reagents capable of recognizing the analyte having different Raman tag selective/specific for each analyte.

According to the method for detecting and identifying the presence and/or the amount of at least one analyte in a sample, wherein the Raman tag selective/specific for the analyte is recognized by the corresponding bacteria what increases the intensity of the SERS spectrum.

In another aspect, the invention relates to the use of a kit comprising the surface-enhanced Raman scattering-(SERS)-active reagent of the invention in a method for detecting, identifying and/or quantifying an analyte.

In another aspect, the invention relates to a method for synthesizing the reagent of the invention comprising the steps of:
a) providing particles functionalized for binding nanoparticles of step b);
b) adding nanoparticles of a plasmonic material and allowing the immoblilization of a plurality of nanoparticles on the surface of the particles of step a) to form a cluster wherein the surface of the nanoparticles is coated with aliphatic, aromatic and/or polymeric molecules or combinations thereof and at least one Raman label,
c) covering the surface of the cluster with at least one layer of a coating material;
d) optionally, functionalizing the coated material with a compound that allows the immobilization of a reagent capable of detecting an analyte; and
e) adding a reagent capable of detecting an analyte to allow the binding of the reagent to the coated cluster.

All the particular embodiments of the reagent of the present invention are also applicable to the method for synthesizing the reagent of the invention.

Step a) of the method of the invention comprises providing particles functionalized for binding nanoparticles of step b).

The particle a) may be a silica, titania, polystyrene, melamine, latex or other rubber microspheres or magnetic polystyrene particle. In a preferred embodiment, the particle a) is a polystyrene particle.

In a preferred embodiment, the particle a) has superparamagnetic properties. In a more preferred embodiment, the particle is a uniform microsphere with superparamagnetic properties (MBs), 1 µm in diameter, with an epoxy functional group.

In another preferred embodiment, the particle a) is a polystyrene particle. In another preferred embodiment, the functionalization is an electrostatic coating, more preferably a stable electrostatic coating. In a more preferred embodiment, the coating of particle a) may be done by covalent or electrostatic (layer-by-layer) binding, by means of an amino, carboxylic acid, epoxy, sulphonate, phosphonate, thiol, chlorosulfonyl, alkene, alkyne and azide functional group, more preferably an amino, carboxylic acid or epoxy functional groups. In a preferred embodiment, the electrostatic coating is performed using polycations and polyanions such as Polyethyleneimine (PEI), Poly(ethylene glycol) (PEG), poly(sodium styrene 4-sulfonate) (PSS), polydiallyldimethylammonium chloride (polyDADMAC), poly(allylamine)-Nafion , poly (acrylic acid)(PAH-Naf/ PAH-PAA), linear N, N-dodecyl,methyl-poly(ethyleneimine), poly (acrylic acid) (DMLPEI/PAA), poly (allylamine hydrochloride) (PAH), poly (allylamine / poly(acrylic acid) (PAH-PAA), poly (acrylic acid) or polyethylene oxide - block - polycaprolactone (PAA/PEO-b-PCL). In a more preferred embodiment the particle a) is functionalized with an epoxy functional group.

In another preferred embodiment, the particle is functionalized by covalent binding. In another preferred embodiment, the functionalization of particle a) is done by electrostatic binding, more particularly by the technique layer by layer.

The functionalization can be performed by any method known in the art, for example by the method disclosed in Mir-Simon, B., et al. Chem. Mater 27(3), 950-958 (2015) and EP3227037A1 and more preferably by the method disclosed in the examples.

In a preferred embodiment the particle a) has a size above 0.3 µm, preferably 0.3 to 8 µm, more preferably 0.3 to 7 µm, more preferably 0.3 to 6.5 µm, more preferably 0.3 to 6 µm, more preferably 0.3 to 5.5 µm, more preferably 0.3 to 5 µm, more preferably 0.3 to 4.5 µm, more preferably 0.3 to 4 µm, more preferably 0.3 to 3.5 µm, more preferably 0.3 to 3 µm, more preferably 0.3 to 2.5 µm, more preferably 0.3 to 2 µm, more preferably 0.3 to 1.5 µm, more preferably 0.3 to 1 µm. In another preferred embodiment the particle a) has a size of 0.3-0.8 µm. In another preferred embodiment the particle a) has a size of 2-3 µm. In another preferred embodiment the particle a) has a size of 3-4 µm. In another preferred embodiment the particle a) has a size of 4-5 µm. In another preferred embodiment the particle a) has a size of 5-6 µm. In another preferred embodiment the particle a) has a size of 6-7 µm. In another preferred embodiment the particle a) has a size of 7-8 µm. In another preferred embodiment the particle a) has a size of 0.3-0.8 µm. In another preferred embodiment, the particle a) has a size of 0.45 µm. In another preferred embodiment the particle a) has a size of 0.5-1 µm. In another preferably embodiment, the particle has a size of 2.5-4.5 µm or 0.5-1 µm; preferably has a size of 2.8-4.5 µm or 0.5-1 µm. In another preferred embodiment, the particle a) has a size of 1 µm. In another preferred embodiment, the particle a) has a size of 0.5 µm. In another preferred embodiment, the particle a) has a size of 2.7 µm. In another preferred embodiment, the particle a) has a size of 2.8 µm. In another preferred embodiment, the particle a) has a size of 4.5 µm.

The size of the particle can be determined by any method known in the art. In a preferred embodiment, the size is determined by microscopy or flow cytometer.

Step b) of the method for synthesizing the reagent of the invention comprises adding nanoparticles of a plasmonic material and allowing the immoblilization of a plurality of nanoparticles on the surface of the particles of step a) to form a cluster wherein the surface of the nanoparticles is coated with aliphatic, aromatic and/or polymeric molecules or combinations thereof, and at least one Raman label.

The immobilization of the plurality of nanoparticles coated with aliphatic, aromatic and/or polymeric molecules or combinations thereof and at least one Raman label on the surface of the particle a) can be achieved by several methods known in the art.

"Cluster" as used herein relates to an aggregation formed by a core of a particle having a shell of a plurality the nanoparticles on the surface of said particle.

In a preferred embodiment, the nanoparticles are synthesized by the method disclosed in Mir-Simon, B., et al. Chem. Mater 27(3), 950-958 (2015) and EP3227037A1 and in the examples of the present invention.

In a preferred embodiment, the plasmonic material is selected from the group consisting of gold, silver, copper, palladium, rhodium, aluminium, ruthenium, indium, alkaline, alkaline-earth metals, metals alloys thereof, CuSe, CuTe, silicon and the combinations thereof, particularly silver or gold. In a particular embodiment, the plasmonic material comprises, but is not limited to gold, silver, or copper, aluminium, or alloys thereof, or a combination thereof. In a specific, non-limiting example, the plasmonic material is gold, silver or copper. In a more preferred embodiment, the plasmonic material is silver.

The surface of the nanoparticle is coated with aliphatic, aromatic and/or polymeric molecules or combinations thereof, and at least one Raman label.

In a preferred embodiment, the aliphatic, aromatic and/or polymeric molecule is selected from the group consisting of organic dyes, organometallic compounds, aromatic compounds, polymeric compounds such as poly(ethylene glycol) (PEG), polyethyleneimine (PEI), poly(sodium styrene sulfonate), 1-ethenylpyrrolidin-2-one (PVP), polyelectrolytes or detergents such as cetyltrimethylammonium bromide (CTAB) and anionic poly(sodium 4-styrenesulfonate) (PSS), thiolated molecules such as mercaptoundecanoic acid or mercaptopropanoic acid, 6-mercaptohexanoic acid, 4-mercaptobutyric acid, 8-mercaptooctanoic acid, 12-mercaptododecanoic acid, 16-mercaptohexadecanoic acid, catechol compounds such as 3,4-Dihydroxyhydrocinnamic acid, 3,4-dihydroxyphenylacetic acid, caffeic acid, dopamine, 3,4-Dihydroxy-L-phenylalanine, DL-Norepinephrine hydrochloride and combinations thereof.

In a preferred embodiment, the at least one Raman label is selected from the group consisting of organic dyes, organometallic compounds and aromatic compounds.

Preferably, the Raman label is selected from the group consisting of 2-mercaptopyridine; benzenethiol; 4- mercaptobenzoic acid; 4-nitrobenzenethiol; 3,4-dicholorobenzenethiol, 3-fluorothiophenol; 4-fluorothiophenol; 3-5-bis(trifluoromethyl)benzenethiol; methylene blue; nile blue A; rhodamine 6G; Toluidine Blue O, 2-Phenylethanethiol, 4-Mercaptophenol, Biphenyl-4-thiol, 7-Mercapto-4-methylcoumarin, 4-(Hydroxyphenyl)-1H-tetrazole-5-thiol, 2-Fluorothiophenol, Crystal Violet, 2-Naphthalenethiol, 4-(((3-Mercapto-5-(2-methoxyphenyl)-4H-1,2,4-triazol-4-yl)imino)methyl)phenol, (2-Trifluoromethyl)benzenethiol, 4-Aminothiophenol, 1-Naphthalenethiol, 1,1',4,1"-Terphenyl-4-Thiol, Biphenyl-4,4'-dithiol, Thiosalicylic acid, 4-(((3-Mercapto-5-(2-pyridinyl)-4H-1,2,4-triazol-4-yl)imino)methyl)-1,2-benzenediol, 4-(((3-Mercapto-5-(2-pyridinyl)-4H-1,2,4-triazol-4-yl)imino) methyl)benzoic, 2,3,4,6-Tetrafluorobenzenethiol, and (5-(4-Methoxyphenyl)-1,3,4-oxidazole-2-thiol).

In a preferred embodiment, the size of the nanoparticles is between 5 to 200 nm, preferably 10 to 180 nm, more preferably 15 to 175 nm, more preferably 20 to 170 nm, more preferably 25 to 165 nm, more preferably 30 to 160 nm, more preferably 35 to 155 nm, more preferably 40 to 150 nm, more preferably 45 to 145 nm, more preferably 50 to 140 nm, more preferably 55 to 135 nm, more preferably 60 to 130 nm, more preferably 65 to 125 nm, more preferably 70 to 120 nm, more preferably 75 to 115 nm, more preferably 80 to 110 nm, more preferably 85 to 100 nm. In another preferred embodiment, the size of the nanoparticles is between 30-90 nm, more preferably the size of the nanoparticles is between 60-70 nm. In a more preferred embodiment, the size of the nanoparticles is between 60-75 nm. In a more preferred embodiment, the size of the nanoparticles is 70 nm. In another more preferred embodiment, the size of the nanoparticles is 60 nm. In another more preferred embodiment, the size of the nanoparticles is 65 nm. In another preferred embodiment, the size of the nanoparticles I between 30-55 nm, more preferably 30 to 50 nm, more preferably 30-45 nm, more preferably 30-35 nm, more preferably 35-40 nm, more preferably 40-45 nm, more preferably 40-55 nm, more preferably 45-55 nm, more preferably 45-50 nm, more preferably 50-55 nm.

The definitions and embodiments in relation to particle size are accordingly applicable to nanoparticle size.

In a preferred embodiment, the size of the nanoparticle according to the invention correspond to the size determined by microscopy TEM and UV-vis spectroscopy ......

Step c) of the method for synthesizing the reagent of the invention comprises covering the surface of the cluster with at least one layer of a coating material;.

In a preferred embodiment, the coating material is selected from the group consisting of a metal oxide, a polymer, a protein, a polysaccharide and a lipid. In a more preferred embodiment, the metal oxide is selected from the group consisting of silicon dioxide, titanium dioxide, aluminium oxide, cerium oxide, zinc oxide, and zirconium oxide. In a preferred embodiment, the coating material is silicon dioxide, also known as silica, or titanium dioxide, also known as titanium(IV) oxide or titania.

The thickness of the coating may vary. By illustrative, and without wishing to be limiting in any manner, the thickness of the coating may be applied in a controlled manner over the core Raman active reporter molecules and the aliphatic, aromatic and/or polymeric molecule or combinations thereof. The thickness of the coating, once complete, may be about 1 nm and 400 nm, or any value there between; for example, the silica or titania coating may be about 1, 10, 20, 25, 30, 35, 40, 45, 50, 55, 60, 65, 70, 75, 80, 85, 90, 95,100, 150, 200, 250, 300, 350 or 400 nm thick, or any value there between. In a preferred embodiment, the layer c) of coating material has a thickness of 1 to 300 nm; preferably from 1 to 120 nm. Several methods known in the art can be used to determine the thickness of the coating. In a preferred embodiment, the thickness of the coating is determined by transmission electron microscopy (TEM).

The coating can be performed by any method known in the art, for example the silica coating can be performed by a modified Stober procedure Mir-Simon, B., et al., cited supra, and Stöber, W., et al. J. Colloid Interface Sci. 26, 62-69 (1968), as disclosed in the examples. The titania coating can be performed by the method disclosed in (J. Am. Chem. Soc. 2012, 134, 11864-11867; Nanotechnology 24 (2013) 415601) and in the examples.

The coating material used according to the invention must be transparent for Raman signal and/or compatible with Raman signal that is, the imaginary component of the refractive index of the material is zero. The transparency of a material can be determined by Raman spectroscopy.

Step d) of the method for synthesizing the reagent of the invention comprises optionally, functionalising the coated material with a compound that allows the immobilization of a reagent capable of detecting an analyte.

In a preferred embodiment, the compound capable of detecting an analyte is selected from the group consisting of an antibody or functional fragments thereof, aptamers, enzymes, nucleic acids, and peptides. In a more preferred embodiment, the compound capable of detecting an analyte is an antibody.

In case that the coated material further comprises a compound that allows the immobilization of the reagent capable of detecting an analyte and said reagent is an antibody, the compound can be selected from the group consisting of silanes, phosphines, thiols, carboxylic acids, amines, epoxides, and catechols, more preferably a silane, more preferably the silane is selected from the group consisting of (Aminopropyl)trimethoxysilane (APTMS), 3-Glycidyloxypropyl trimethoxysilane (GPTMS) and 3-Mercaptopropyl trimethoxysilane (MPTMS). In another preferred embodiment, the compound that allows the immobilization of the reagent capable of detecting an analyte is phospine, more preferably is 2-aminoethyl dihydrogen phosphate. In another preferred embodiment, the compounds that allow the immobilization of the reagent capable of detecting an analyte is a catechol, preferably Dopamine hydrochloride, 3,4-Dihydroxyphenethylamine hydrochloride. In another preferred embodiment, the reagent capable of detecting an analyte is a thiol.

Step e) of the method for synthesizing the reagent of the invention comprises adding a reagent capable of detecting an analyte to allow the binding of the reagent to the coated cluster.

This step can be performed by electrostatic or covalent adsorption, as previously described. In a preferred embodiment, covalent immobilization of the reagent capable of detecting an analyte is preferred, using carbodiimide reactions to obtain peptide bonds (Pazos-Perez, N., et al. Sci. Rep. 6, 29014 (2016), Catala, C., et al. Adv. Mater. Technol. 1 (8), 1600163 (2016)). In another preferred embodiment the compound that allows the immobilization of a reagent capable of detecting the analyte is very reactive, and thus it directly links to the reagent capable of detecting the analyte. This is the case for example of the epoxy group of GPTMS which is directly bound to antibodies. Standard protocols can be followed in this case (Dynabeads^{™}, BangsLab Protocols).

All the particular embodiments of the reagent of the present invention are also applicable to these aspects of the invention.

The invention is defined by the following aspects:
1. A surface-enhanced Raman scattering-(SERS)-active reagent, comprising in order:
   a) a core of a particle functionalized for binding of element b);
   b) a shell of a plurality of nanoparticles of a plasmonic material immobilized on the surface of the particle a) wherein the surface of the nanoparticles is coated with aliphatic, aromatic and/or polymeric molecules or combinations thereof, and at least one Raman label,
   c) at least one layer of a coating material covering element b) that interacts with element d); optionally, the layer of the coating material is functionalized with a compound that allows the immobilization of a reagent capable of detecting an analyte; and
   d) a reagent immobilized on the coating layer c) capable of detecting an analyte.
2. The SERS active reagent according to aspect 1, wherein the coating material further comprises a compound that allows the immobilization of the reagent capable of detecting an analyte and/or the coating material is modified to interact with element d).
3. The SERS active reagent according to any one of aspects 1 or 2, wherein the particle a) has a size above 0.3 µm, preferably 0.3 to 8 µm, more preferably 0.3 to 4.5 µm, more preferably 0.3-0.8 µm, more preferably 2.5-4.5 µm or 0.5-1 µm.
4. The SERS active reagent according to any one of aspects 1 to 3, wherein the functionalization of particle a) is done by covalent or electrostatic (layer-by-layer) binding, by means of an amino, carboxylic acid, epoxy, sulphonate, phosphate, thiol, chlorosulfonyl, alkene, alkyne and azide functional group, more preferably an amino, carboxylic acid or epoxy functional groups.
5. The SERS active reagent according to any one of aspects 1 to 4, wherein the particle has superparamagnetic properties.
6. The SERS active reagent according to any one of aspects 1 to 5, wherein the size of the nanoparticles is 5 to 200 nm, preferably 30-90 nm.
7. The SERS active reagent according to any one of aspects 1 to 6, wherein the plasmonic material is selected from the group consisting of gold, silver, copper, palladium, rhodium, aluminium, ruthenium, indium, alkaline, alkaline-earth metals, metals alloys thereof, CuSe, CuTe, silicon and the combinations thereof, particularly silver or gold.
8. The SERS active reagent according to any one of aspects 1 to 7, wherein the aliphatic, aromatic and/or polymeric molecule is selected from the group consisting of organic dyes, organometallic compounds, aromatic compounds, and polymeric compounds, preferably poly(ethylene glycol) (PEG), polyethyleneimine (PEI), poly(sodium styrene sulfonate), 1-ethenylpyrrolidin-2-one (PVP), polyelectrolytes or detergents such as cetyltrimethylammonium bromide (CTAB) and anionic poly(sodium 4-styrenesulfonate) (PSS), thiolated molecules such as mercaptoundecanoic acid or mercaptopropanoic acid, 6-mercaptohexanoic acid, 4-mercaptobutyric acid, 8-mercaptooctanoic acid, 12-mercaptododecanoic acid, 16-mercaptohexadecanoic acid, catechol compounds such as 3,4-Dihydroxyhydrocinnamic acid, 3,4-dihydroxyphenylacetic acid, caffeic acid, dopamine, 3,4-Dihydroxy-L-phenylalanine, DL-Norepinephrine hydrochloride and combinations thereof.
9. The SERS active reagent according to any one of aspects 1 to 8, wherein the at least one Raman label is selected from the group consisting of organic dyes, organometallic compounds and aromatic compounds.
10. The SERS active reagent according to aspect 9, wherein the organic dye is selected from the group consisting of rhodamines, indole derivatives, azo dyes, porphyrins, perylenes, acridines, anthraquinones, arylmethanes, indamines, oxazones, oxazines, indophenols, thiazines, xanthens and fluorenes; the organometallic compound is selected from the group consisting of porphyrins, phthalocyanines and organic lacquers coordinated with transition metals; and/or the aromatic compound is selected from the group consisting of thiolated, aminated or nitrated derivatives of the benzene, naphthalene, pyrene, anthracen, benzenethiol and naphthalenethiol.
11. The SERS active reagent according to any one of aspects 1 to 22, wherein the coating material is selected from the group consisting of a metal oxide, a polymer, a protein, a polysaccharide and a lipid.
12. The SERS active reagent according to aspect 11, wherein the metal oxide is selected from the group consisting of silicon dioxide, titanium dioxide, aluminum oxide, cerium oxide, zinc oxide, zirconium oxide; the polymer is selected from the group consisting of polyethylene glycol (PEG), sulfonated polystyrene (SPS), polystyrene (PS), styrene, acrylate derivatives, maleimide, polyether ether ketone (PEEK), polyvinylpyrrolidone (PVP), epoxy resins, diglycidyl ether of bisphenol A (DGEBA), poly(butyl acrylate), saturated fatty acids such as lauric acid, myristic acid, palmitic acid, and stearic acid, and unsaturated variants thereof, such as palmitoleic acid, oleic acid, linoleic acid, and linolenic acid; the protein is selected from the group consisting of bovine serum albumin and casein; the polysaccharide is selected from the group consisting of cellulose, dextran, glycogen, chitin, galactogen and starch; and/or wherein the lipid is an aliphatic lipid.
13. The SERS active reagent according to aspect 10, wherein the coating material is a metal oxide, as silicon dioxide or titanium dioxide.
14. The SERS active reagent according to any one of aspects 1 to 11, wherein the layer c) has a thickness of 1 to 300 nm; preferably from 1 to 120 nm.
15. The SERS active reagent according to any one of aspects 1 to 14, wherein the reagent capable of detecting an analyte is selected from the group consisting of an antibody or functional fragments thereof, aptamers, enzymes, nucleic acids, and peptides.
16. The SERS active reagent according to any one of aspects 1 to 15, wherein the reagent capable of detecting an analyte is an antibody.
17. The SERS active reagent according to aspect 16, wherein the compound that allows the immobilization of the antibody is selected from the group consisting of silanes, phosphines, thiols, carboxylic acids, amines, epoxides, and catechols.
18. The SERS active reagent according to aspect 17, wherein the silane is selected from the group consisting of Aminopropyl)trimethoxysilane (APTMS), 3- Glycidyloxypropyl trimethoxysilane (GPTMS) and 3-Mercaptopropyl trimethoxysilane (MPTMS); the phosphine is 2-aminoethyl dihydrogen phosphate (AEPH2); the thiol is mercaptoundecanoic acid and mercaptopropionic acid, and/or the catechol is dopamine.
19. The SERS active reagent according to any one of aspects 1 to 18, wherein the analyte is a microorganism or a mammalian cell.
20. A method for detecting, identifying and/or quantifying an analyte in a sample, which comprises the use of the SERS active reagent according to any one of aspects 1 to 19.
21. A method for detecting and identifying the presence and/or the amount of at least one analyte in a sample, the method comprising:
   a) incubating the sample comprising the analyte with a reagent capable of recognizing the analyte, during a time sufficient to allow the formation of a complex between the reagent and the analyte;
   b) separating the complex of the reagent and the analyte from the sample;
   c) optionally, staining the analyte with a fluorescence label;
   d) eluting the analyte from the complex
   e) incubating the eluted analyte, with at least one Raman tag selective/specific for the analyte during a time sufficient to allow the labelling of the eluted analyte with the selective Raman tag; and
   f) acquiring an image of the sample with an optical and/or fluorescence microscope and
   g) measuring Raman spectroscopy to detect and identify the presence and/or amount of the at least one analyte.
22. The method according to aspect 21, wherein the sample is a fluid sample.
23. The method according to aspect 22, wherein the fluid sample is selected from the group consisting of blood, serum, plasma, ascites, pus, synovial fluid, peritoneal fluid, amniotic fluid, lymph fluid, pericardial fluid, cervicovaginal fluid, urine, semen, sputum, cerebral spinal fluid, tears, mucus, sweat, milk, brain extracts and saliva, preferably blood
24. The method according to any one of aspects 21 to 23, wherein the sample is diluted before step a), preferably the sample is diluted 1:1, more preferably the sample is diluted in PBS or saline solution.
25. The method according to any one of aspects 21 to 24, wherein the reagent in step a) comprises a particle, preferably a magnetic particle.
26. The method according to any one of aspects 21 to 25, wherein the reagent in step a) comprises an antibody or fragments thereof, aptamers, enzymes, nucleic acids, or peptides, or any combinations thereof.
27. The method according to any one of aspects 21 to 26, wherein the selective Raman tag is a SERS tag according to any one of claims 1 to 19.
28. A method for synthesizing the reagent of any one of aspects 1 to 19 comprising the steps of:
   a) providing particles functionalized for binding nanoparticles of step b)
   b) adding nanoparticles of a plasmonic material and allowing the immoblilization of a plurality of nanoparticles on the surface of the particles of step a) to form a cluster, wherein the surface of the nanoparticles is coated with aliphatic, aromatic and/or polymeric molecules and at least one Raman label, or combinations thereof;
   c) covering the surface of the cluster with at least one layer of a coating material;
   d) optionally, functionalizing the coated material with a compound that allows the immobilization of a reagent capable of detecting an analyte; and
   e) adding a reagent capable of detecting an analyte to allow the binding of the reagent to the coated cluster.
29. The method according to aspect 28, wherein the particle has a size above 0.3 µm, preferably 0.3 to 8 µm, more preferably 0.3 to 4.5 µm, more preferably 2.5-4.5 µm or 0.5-1 µm.
30. Use of the surface-enhanced Raman scattering-(SERS)-active reagent according to any one of aspects 1 to 19 in a method for detecting, identifying and/or quantifying an analyte.
31. A kit comprising the surface-enhanced Raman scattering-(SERS)-active reagent according to aspects 1 to 19.
32. Use of the kit according to aspect 31 in a method for detecting, identifying and/or quantifying an analyte.

This invention is further illustrated by the following examples, which are not to be construed in any way as imposing limitations upon the scope thereof.

### Examples

### MATERIALS AND METHODS

### SERS Tags production

Figure 1 and Figure 2 show schematic representations of the general composition of the developed SERS Tag (an example of SERS active reagent) and their synthetic procedure, respectively.

Normally, the core particle is a commercial microparticle, such as silica, titania, polystyrene, melamine, latex or other rubber microspheres, or magnetic polystyrene microspheres (superparamagnetism properties). These commercial particles can be obtained from diverse providers, such as Dynabeads^{™}, BangsLabs, Microparticles GmbH, Cospheric, NanoComposix, LiStarFish, among others. The core diameter of the particle can range from 0.3 µm up, such as from 0.3 µm to 5 µm; and their surface can be covered with different functional groups, such as epoxy, carboxyl, or amine groups. Although the fabrication procedure for SERS Tags is the same in all cases, the size should be taken into account for stoichiometry in conjugation step and for the addition of the different reagents during the whole synthesis. Cleaning of the particles is necessary after each step of the procedure to remove the excess of reagents. In case of magnetic core microparticles, one can take advantage of the magnetic properties of the microspheres to concentrate the sample using a neodymium magnet and remove the supernatant. In case of polystyrene, melamine or silica core microparticles one should employ a centrifuge to concentrate particles in a pellet. In both cases, the particles are redispersed in the appropriate volume of solvent (water or ethanol).

The preparation of the SERS Tags using 1 µm-diameter magnetic microparticles with an epoxy functional group is described next, which in this case starts with a layer-by-layer process. Different polyelectrolytes can be used such as polyethyleneimine (PEI), poly(ethylene glycol) (PEG), poly (acrylic acid) (PAA), or poly(sodium styrene 4-sulfonate) (PSS). Depending on the microparticle functional group, the order of the polyelectrolyte used must be changed. For epoxy microparticles, the layer-by-layer process starts with the addition of PEI, a positively charged polyelectrolyte, followed by the addition of PSS, a negatively charged polyelectrolyte. Many examples from the literature can be used (ACS Nano 2019, 13, 6, 6151-6169). For instance, 50 mg of 1 µm-diameter epoxy magnetic microparticles are suspended into 25 mL of a 2 mg mL⁻¹ PEI aqueous solution, previously sonicated 30 min. The mixture is stirred for 2 h and then washed twice with 25 mL Milli-Q water using a magnet. Same procedure is applied for addition of PAA, or any other additional polyelectrolyte layer. Finally, microparticles are redispersed in 4-5 mL of Milli-Q water and their concentration is measured using a flow cytometer equipment.

Once microparticles have an electrostatic coating, metallic nanoparticles with Raman labels are conjugated to their surface. Spherical silver nanoparticles of 60-75 nm are employed in this example. The synthesis of the colloid is disclosed in Mir-Simon, B., et al. Chem. Mater 27(3), 950-958 (2015) and EP3227037A1. Briefly, 5 mL of a previously prepared solution of 5 mL Na₃Cit (0.164 M) + 600 µL ascorbic acid (0.1 M) is added to 500 mL of boiling water under vigorous stirring. Then, 2.605 mL of a prepared solution of 2.230 mL AgNOa (0.1 M) + 1.680 mL MgSO₄ (0.1 M) are added to the mixture. The solution is left for 30 min under stirring and temperature, leaving the mixture to cool down to room temperature. Colloidal cleaning is done by two centrifugations at around 5200 rpm for 15 min (Centrifuge, Hettich zentrifugen, Universal 320-R). Supernatant is removed, and volume is restored to 7 mL of MilliQ water. The synthesized silver nanoparticles (AgNPs) can be characterized by UV-vis spectroscopy and TEM microscopy. The functionalization reaction consists in a EtOH/AgNPs (1:1) mixture with the Raman label and the stabilizer. The necessary amount of reagents will depend on the Raman label used and the molecules/nm². As example, 69.8 mL of 3×10¹⁰ NPs/mL and 69.8 mL of ethanol are mixed with a mixture of 107 µL MUA (10⁻³ M), 322 µL of 4-MBA (10⁻³ M) and 25 µL of NH₄OH at 30%. The mixture is left overnight under stirring. Cleaning is performed by two centrifugations at around 4800 rpm for 15 min. Functionalized AgNPs are characterized by UV-vis spectroscopy, TEM and Raman spectroscopy to check their Raman signal.

Once nanoparticles are functionalized with the Raman label, they can be used to cover the charged microparticles. Since these nanoparticles are also charged, they can remain electrochemically attached to the surface of the microparticles. A complete coating of these nanoparticles on the surface of the microparticle is preferred to ensure a high Raman signal. The higher the signal can be, the more efficient SERS Tags will be obtained, which is normally related with the analysis time of samples. Theoretical calculations using basic geometry allow to know the number of nanoparticles which can cover a microparticle. For example, for a 1 µm-diameter microparticle it is necessary an amount of 260 spherical 70 nm nanoparticles. Normally, microparticles are in default compared to nanoparticles, ranging from 50-100%. For this example, 40 mL of a solution of silver nanoparticles [Ag] = 3·10¹⁰ NP/mL should be added to 3.5 mL of a solution of 1.5·10⁹ microparticles/mL. The mixture is kept under stirring at least 2 h for conjugation. After that time, the suspension is washed twice with Milli-Q water. At this point, the structure has an intrinsic intense Raman signal, since molecules coating the surface of the nanoparticles are close to the surface of plasmonic nanoparticles.

To ensure a constant and uniform signal, the cluster can be protected with at least one layer of an external coating with a coating material, which should be transparent in Raman signal to maintain the SERS properties of the nanoparticles, such as silica or titanium dioxide. This coating preserves leaching of the code, maintaining the SERS properties, and also avoids from other contamination from other Raman compounds. Additionally, the coating is functionalized with molecules with different functional groups, which make possible the immobilization of a biorecognition element (a reagent capable of detecting an analyte), such as antibodies. Coating of the structure can be carried out through silica or titania precursors. Figure 2 shows silica coating particles, carried out by a modified Stober procedure Mir-Simon, B., et al., cited supra, and Stöber, W., et al. J. Colloid Interface Sci. 26, 62-69 (1968). Thus, tetraethyl orthosilicate alkoxide (TEOS, silica precursor) is added to the conjugated microparticles in ethanol under basic conditions (NH₄OH), leaving the mixture shaking to achieve its hydrolysis and condensation. Silica hardening can be also carried out by heating the cluster solution at 60-140°C overnight in a silicone bath. The shell layer can be confirmed by TEM, which thickness can be calculated using image measurement tools. For this example, 1.65 mL of a 1×10⁹ conjugated microparticles/mL solution are placed in 32.52 mL of EtOH and 3.35 mL of Milli-Q water and sonicated. Then, 662 µL of NH₄OH, 30%, and 17.8 µL of TEOS, 100%, are added and left under agitation for at least 2 h. The silica coated clusters are cleaned twice with 25 mL of EtOH. The procedure can be repeated (such as three times) to obtain a thicker silica layer.

Silica can be modified or functionalized with other molecules to provide functionality to the surface of the cluster, which allows the immobilization of a reagent capable of detecting an analyte, such as an antibody. Silanes such as (3-aminopropyl)trimethoxysilane (APTMS) or (3-glycidyloxypropyl)trimethoxysilane (GPTMS); catechols such as dopamine; thiols such as 6-amino-1-hexanethiol hydrochloride; or phosphines such as 2-aminoethyl dihydrogen phosphate, can be used in this step. Molecule selection will depend on the type of biofunctionalization to perform. An excess of the reagent is normally employed to ensure a complete covering of the surface, such as 1 to 10⁶ ratios. Once selected the desired ratio, the cluster solution is mixed with the appropriate volume of the reagent and left overnight for incubation in ethanol or water under stirring. Size and aggregation of the cluster can be checked again by TEM. In this case, 1×10⁸ silica coated clusters in 1.75 mL of EtOH are mixed with 64 µL of a 1 mg/mL of dopamine. The solution is incubated overnight at 60°C. Excess of reagents are cleaned twice using 2 mL of EtOH. Finally, the solution is heated overnight at 90°C for silica hardening. Functionalized clusters can be resuspended in EtOH for the necessary concentration.

Titania coating can also provide stability to the cluster. In this case, titanium (IV) isoproxide (TTIP) or tetrabutyl titanate (TBOT) can be employed as a precursor, which can be used under basic conditions (ethanol/ammonia mixtures) or dissolved in an organic solvent or a mixture such as ethylene glycol in acetone, as described in the literature (J. Am. Chem. Soc. 2012, 134, 11864-11867; Nanotechnology 24 (2013) 415601). In both cases, the precursor allows the formation of titania nanoparticles when the mixture is added to the conjugated microparticles.

In the example shown, 45 µL of TTIP, 0.15 mmol, are added to 10 mL of ethyleneglycol and shacked overnight under agitation. 5.5×10⁸ conjugated microparticles are dispersed in 10 mL of acetone and 0.4 mL of water. The previous 10 mL mixture is added to the cluster suspension and the resulted solution is shacked for 2 h. The solution was removed from the titania coated clusters and 35 mL of water were added. Then, the solution is heated at 100°C for 4 h. The titania coated clusters can be cleaned again with EtOH and water until the solution is completely cleaned, and resuspended in Milli-Q water for the necessary concentration.

As for silica coating, titania coated clusters can be functionalized to provide functional groups to the external layer of the SERS reagent. In this case, phosphoamines such as 2-aminoethyl dihydrogen phosphate, catechols such as dopamine can be used, among other compounds. The experimental procedure used is equivalent to the previous described. As before, TEM microscopy can confirm the size of the developed clusters. In this example, 3×10⁸ titania coated clusters redispersed in 1.75 mL of water are mixed with 192 µL of a 1 mg/mL of dopamine. The mixture is shaken overnight at 60°C. Functionalized clusters are cleaned twice in water.

Finally, the developed SERS clusters or SERS Tags need to be biofunctionalized with a biorecognition element, which can be done by electrostatic or covalent adsorption. Covalent immobilization of the biorecognition element is preferred, being feasible to use different standardized protocols depending on the functional group which has been used in the functionalization of the SERS Tags, and depending on the biorecognition element. For instance, in case of functionalization of SERS Tags with carboxylic acid or amine functional groups, the well-known carbodiimide reaction can be employed to obtain peptide bonds (Sci. Rep. 6, 29014 (2016); Adv. Mater. Technol. 1 (8), 1600163 (2016); Dynabeads^{™}, BangsLab Protocols). To minimize non-specific adsorptions during the biochemical assay, such as cell debris or blood proteins, their surface is blocked with buffer solutions containing proteins and/or surfactants, such as BSA, casein and/or Tween 20^{®}, Tris, among others. Flow cytometry analysis (NovoCyte, Acea Bioscience, APC filter) confirm the correct biofunctionalization, where the biofunctionalized SERS Tags are conjugated to a secondary antibody labelled with a fluorophore (Dye 647) using standard protocols. As negative controls, SERS Tags incubated with a secondary antibody with reactivity for different host, and SERS Tags without any secondary antibody. Since the whole external layer has been functionalized, a homogeneous and complete coating with the biorecognition element can be attained, which enhances the sensibility of SERS Tags for the analyte. The size of the cluster can also help in this issue, which can be tunned depending on the core microparticle used. Selectivity and sensibility of the SERS Tag can improve when it is adequate to the analyte size. In a typical biofunctionalization, 5×10⁷ titania coated clusters functionalized with dopamine, previously cleaned in PBS1x, are mixed with 72 µL of 28 mM NHS, 4.35 µL of 50 mM EDC, 26.85 µL of 1.1 mg/mL IgM antibody solution, and 396 µL PBS1x at pH 7.4. The mixture is incubated overnight under agitation at 4°C. The solution is cleaned three times with PBS1x. Biofunctionalized SERS Tags are incubated in 1 mL of a blocking buffer solution for 2 h and washed again three times in PBS. The blocking buffer solution is composed of 0.5-5% BSA at pH 7.

As previously described, different characterization techniques are used during the SERS Tags production. Details are described as follows. For TEM characterization (TEM, JEOL 1011operating at 80 kV), a drop of 20 µL of a 1×10⁵ - 1×10⁷ particles /mL solution is placed on a TEM grid and let completely dry. All examples shown in figures have been done using a concentration of around 1×10⁶ beads /mL. Morphology, average size, and polydispersity of at least 100 particles are checked using "Image J" software.

For UV-vis characterization, spectra are recorded from 300 to 900 nm with a Thermo Fisher Scientific Evolution 201. For nanoparticles Raman spectroscopy characterization, an inVia Raman equipment from Renishaw is employed, with an excitation laser of 785 nm and 300 mW, with a x10 objective, 0.5s, 1 acquisition, 1200 cm⁻¹ centred. Colloidal solutions are normally in a concentration range of 1 × 10⁹ - 1 × 10¹² NPs/mL, typically, 1 × 10¹⁰. For SERS characterization of clusters, a concentration of 1 × 10⁶ beads /mL is used, placing 5 µL into a glass slide. The SERS signal is measured with a RA816 equipment (Renishaw plc), with an excitation laser of 785 nm, a x50 objective, LD, 25% laser power, and 0.497 s exposure time, acquiring various maps (with intensity and similarity spectra) to obtain measurements of at least 20-25 single beads. Each spectrum comes from the pixel more intense of the resulting intensity cluster of each bead. For flow cytometry characterization, concentrations from of 1×10³ - 5×10⁸ beads/mL can be used. Normally, 1×10⁵ - 5×10⁶ beads/mL are preferred.

### Biochemical assay

Figure 10 shows a schematic representation of the biochemical assay using the developed SERS Tags. As it can be observed, it consists of different steps. In this case, the example is addressed to the detection of a microorganism, such as a fungus, in blood.

To do so, a small volume of blood (0.5 - 1mL, collected in a sodium citrate tube) is diluted with PBS or saline solution, normally 1:1, and mixed with a reagent capable of recognizing the analyte, such as well-known polystyrene microspheres. This commercial reagent is biofunctionalized with a biorecognition element, like antibodies or aptamers, and it is capable of recognizing at least the target microorganism. In this case, magnetic particles of 1.0 - 4.5 µm biofunctionalized with an antibody have been employed. Their core is composed of ferrite and/or magnetite nanoparticles which are embedded in a polystyrene matrix (superparamagnetism properties). For the biofunctionalization, recommended protocols from the commercial supplier have been followed (User Guides, Dynabeads^{™}, BangsLab). Many types of particles can be used, such as those functionalized with epoxy, tosyl-activated, carboxyl, or amine groups, or even those with streptavidin, protein A, or protein G, among others. The addition of certain additives, such as SPS, saponin or surfactants, may be necessary. The use of the SPS favours the growth of certain microorganisms, which are susceptible to components present in the blood. Saponin or other surfactants breaks blood cells and/or permeabilize microorganisms. In this example, no additive was used. The polystyrene microspheres volume and concentration for the incubation may vary. In any case, recommended protocols from the commercial supplier (Dynabeads^{™}, BangsLab) have been followed. As example for 2.8 µm-sized beads, 1-10 × 10⁸ beads/mL is the recommended range concentration of beads (5mg beads/20µg protein). Incubation can be performed from 4°C to 37°C under shaking during 10 min to 4 h. In this example, incubation should take place at 37°C for 1 h. Thereafter, the sample is cleaned by means of a magnet, removing the cell matrix and keeping the complex microorganism-microsphere. A neodymium magnet is used, which attracts all magnetic microspheres, concentrating them in a point of the vessel, allowing to remove the liquid that contains the matrix. To thoroughly clean the sample, several washes using a saline or PBS solution may be required following the process described. Some surfactants such as Tween20^{®} or Brij 30^{®} may be employed.

Before isolating the microorganism from the microsphere, staining of the analyte can be performed using a dye or fluorophore. Different compounds can be employed, such as cell surface, DNA, live and dead cells, or intracellular structures staining. Some examples can be DAPI, CalcoFluor White, Direct Red 23, Alexa compounds, etc. Alternatively, the staining could be done at the end of the assay, including the option of incubating the dye/fluorophore with the SERS Tags. In this case, the dye/fluorophore should not present any adsorption in the SERS Tags to avoid their labelling. Normally, it is necessary to remove the excess of dye. However, the use of some specific fluorophores, which only stain specific structures, can avoid this step, such as in the case of calcofluor white. In this example, a nucleic acid fluorophore that emits in the orange is used, SYTO^{™} 83 Orange Fluorescent Nucleic Acid Stain. A small volume of this fluorophore is added to the sample, at a concentration of 10-20 µM (recommended protocol). To ensure labelling of microorganisms, the sample is incubated for some time, 10 - 60 min, at room temperature or 37°C.

Next step is addressed to the elution of the target from the microsphere. Different strategies can be used, which specific conditions can be checked in commercial protocols.

A common option is the use of low or high-pH buffers, which dissociates the antibody from the immobilized analyte. Glycine-HCl at pH 2.5, citric acid at pH 3.0, triethylamine, glycine or triethanolamine at pH 11.5, or ammonium hydroxide at pH 10.5 are some typical examples. pH and concentration should be optimized for each specific application. Other strategies rely on increasing the ionic strength and/or the use of chaotropic effects, such as high concentration of magnesium chloride, lithium chloride, sodium iodide, or sodium thiocyanate. Protease inhibitors as papain, pepsin, ficin, or trypsin are another alternative for antibodies fragmentation, as well as disulphide reducing agents, like dithiothreitol (DTT) or 2-mercaptoethanol. Immunoprecipitation and/or Western Blot buffers can be also employed, such as RIPA or cell lysis buffers, where detergents are normally included in their composition. In this case, soft conditions are preferred to maintain the analyte structure. Thus, if detergents are used, non-denaturalizing compounds would be preferred, such as non-ionic detergents. Some examples would be NP-40, Brij^{™}30, Triton X-100, or Tween20^{®}. If ionic detergents are used, as sodium dodecyl sulphate (SDS) or sodium deoxycholate (SDC), it would be preferred to employ low concentrations and/or short incubations, such as SDS at a concentration lower than 7%, during few seconds, or for 60 minutes if lower concentration than 7% (PLoS One. 2011 Mar 23;6(3):e18218.). These solutions may include the use of some buffer compound, such as TRIS or PBS; the addition of chelating ligands as EDTA or EGTA; some salts like NaCl, NaF, Na₃VO₄; NaN₃; or any other compound as glycerol, etc. Any combination of them can be also possible. Specific competitors may be also employed. In this case, an excess of the counter ligand or an analogue (≥ 0.1 M) should be used to obtain the exchange of the analyte from the microsphere. For example, in case of streptavidin coated microbeads, where the binding is done through an antibody-biotin complex, a solution of biotin or a streptavidin-Binding Peptide (SBP)-Tag can be employed, where other additional compounds can also be included as co-adjuvants. Equivalent strategies can be used in case of microspheres coated with protein A or protein G. (EP 2 725 359 B1). In most cases, conditioning buffers to restore initial conditions are necessary.

In this case, elution of the microorganism was done using a high-pH buffer, ammonium hydroxide or glycine solution at pH ≥ 10 for at least 5 minutes. A low-pH solution is added to the supernatant once isolated from the microspheres until neutral pH is obtained (pH 6-8). The remaining microsphere pellet can be discarded.

SERS Tags are finally added to the eluted analyte for the selective labelling of the pathogens. At least one type of SERS Tag will be added. Labelling efficiency can depend on the analyte concentration in the sample. If high concentration of analyte is expected, dilution of the sample can be also done. 1-100 µL of 1 ×10³ -1 ×10⁹ SERS Tags / mL can be used in a final sample volume of 10-2000 µL; frequently, 1 ×10⁵ -1 ×10⁷ SERS Tags / mL are used to a final sample volume of 20-1000 µL. Incubation can be performed from 4°C to 37°C under shaking during 10 min to 4 h. In this example, incubation takes place at 37°C for 1 h. Final sample can be concentrated to reduce the total volume. A magnet can be used in case of magnetic SERS Tags, or alternatively, by centrifugation for other core material.

The total assay time would be 2.5 to 3 hours. Figure 10 shows the schematic representation of the different steps of the assay.

### Methodology for the detection and identification of microorganisms. Sample measurement by fluorescence microscopy and Raman spectroscopy

Sample is analysed acquiring white light and/or fluorescence images, and its Raman spectra. A Raman equipment can be used since its usual configuration normally allow white light image acquisition. For fluorescence images acquisition, the Raman microscope should be equipped with a photoluminescence module. Alternatively, white light or fluorescence images and the Raman spectra can be collected separately, with independent and dedicated equipment.

The sample is placed on a solid surface, made preferably of glass, quartz or CaF₂ to minimize Raman background signal. Borosilicate glass supports (glass slides or cover slips), glass fibre or cellulose acetate filters, or thin films of polycarbonate or olefin copolymer can be also employed. The use of inserts or isolators, chamber slides, or perfusion chambers allow to confine the liquid in a specific area. In case of filter measurements, membrane pores should be smaller than 1 µm to retain the analyte on its surface. In case of confined liquid and the use of magnetic SERS Tags, a magnet can help to concentrate the sample in the surface of the material, being feasible even to remove the liquid to measure a dry sample.

The equipment should be provided with a fast and good resolution camera. The faster the camera can work and the bigger area the sensor can collect, the less total analysis time. In case of fluorescence acquisition, the equipment should also include a white light LED excitation source, or dedicated excitation LEDs, and a filter for the selection of the excitation/emission wavelength.

In these examples, an epifluorescence microscope has been used (Nikon Eclipse TS2), which is equipped with a 5 MPixel camera, a CMOS sensor, and a set of ET CY3/TRICT filters. An exposure time of 500 ms has been used for fluorescence images acquisition.

For the Raman spectra acquisition, an excitation laser of 785 nm is used (RA816 Renishaw plc). In any case, other excitation wavelengths such as 532 nm or 633 nm can also be used [Pazos-Perez, N., et al., cited supra, and Álvarez-Puebla, R.A. J. Phys. Chem. Lett. 3, 857-866 (2012)]. This equipment allows the collection of white light images. 10x, 20x, 50x or 100x objectives can be used. All these parameters will impact on the number of photographs required, so in the necessary time to collect images from the whole sample. For example, the use of a 50x objective with a 7 mm × 3.5 mm surface sample require the collection of circa 525 photographs with the described equipment (35x15 photographs, 5-6 minutes acquisition time). Measurement parameters should be optimized for each specific equipment, to collect both white light and/or fluorescence images. Commercial image processing programs such as Image J or Particle Analysis (Renishaw plc) can be useful to analyse images. Dedicated codes or algorithms can be also employed. In the analysis of fluorescent images, the absence of any fluorescence would indicate that there are no pathogens in the sample, while fluorescent images would indicate the presence of microorganisms, confirming thus a blood infection. In this case, the sample should be further scanned for the Raman spectra, which will provide additional information regarding the pathogen identity. Only fluorescent areas are scanned in the Raman measurements, reducing thus the total analysis time. Raman mapping can be done using the highest resolution of the equipment, which in this case is 1 µm. Exposure time and the laser intensity also contribute to achieve a high intense signal but can compromise the integrity of the sample. The high intensity of the developed SERS Tags allows to use low exposure times, which minimizes the analysis time. Data analysis of SERS spectra can generate colour channel images for each of the analysed RL, obtaining a set of images with different channels. Ideally, only one type of RL should be identified in the fluorescent area scanned. The Raman spectrum detected in the SERS measurement can be correlated with the specific antibody immobilized on the surface of the magnetic SERS Tag, making possible the pathogen identification in a multiplex analysis.

The described method is focused to pathogen identification for blood infections, but could be applied to other fluids such as urine, saliva, etc.

### Example 1

Figure 3 shows an example of the magnetic SERS Tags developed. As previously mentioned, these clusters allow the selective identification of different microorganisms in the sample at the time (multiplex), since metallic nanoparticles functionalized with different organic molecules (Raman Labels, RL) are used, which can be correlated to a specific antibody conjugated on the surface of the magnetic SERS Tag.

In this case, TEM images show a cluster with a central magnetic microparticle of 1 µm, which is conjugated with 70 nm silver nanoparticles (left TEM image). The cluster is coated with a silica layer (right TEM image), which has been functionalized with APTMS, and biofunctionalized with a *C. albicans* selective antibody (Dynabeads^{™} M-270 protocol). As the SERS spectrum shows, the synthesized SERS Tag shows an intense signal form the Raman label coating the silver nanoparticle, which in this case corresponds to 4-mercaptobenzoic acid (4-MBA).

### Example 2

Figure 4 shows another example of magnetic SERS Tag. Composition of the cluster is the same as for SERS Tags in Figure 3, but in this case the external coating is titania. Dopamine has been used in this case to functionalise titania shell. As for the previous particles, they show an intense SERS signal. Flow cytometer analysis from biofunctionalized magnetic SERS Tags are also shown. In this case, biofunctionalization was done with a *C. albicans* antibody from rabbit. Secondary antibodies were anti-rabbit from goat as positive sample, and anti-mouse from goat as negative control.

### Example 3

Figure 5 shows a comparison of the Raman signal for clustered silver nanoparticles, where hot spots are formed due to the close contact of nanoparticles, and the developed SERS Tags. As it can be observed, intensity is much higher in case of the developed SERS reagent than in case of silver clusters.

Images and spectra have been collected with an RA816 equipment (Renishaw plc), with the sample was casted on a glass slide. Conditions for acquisition were SL image, 0.45 s exposure time, 25% laser power, 785 nm laser liner, and x50LD objective.

### Examples 4 to 7

Figures 6 to 9 show other examples of SERS Tags with different core diameter and composition, specifically, 0.5 µm polystyrene microparticles, 0.5 µm magnetic microparticles, 2.7 µm magnetic microparticles, and 4.5 magnetic microparticles. All of them have 4-MBA or 4-MPy as Raman label, are titania coated, and have been functionalized with dopamine.

### Example 8- Detection and identification of C. albicans

Next example shows the results obtained in an assay with a blood sample which has been inoculated with a known concentration of *C. albicans.* Two different types of SERS Tags have been used, one biofunctionalized with a *C. albicans* selective antibody, and the other with a *S. aureus* selective antibody. Thus, two different Raman labels identify the described SERS Tags.

- Experimental conditions: For the fungi sample, 1,000 cfu of *C. albicans* has been inoculated to 439 µL of blood, finally diluted to 1 mL. To comparison, a blank sample has been prepared similarly but without fungi inoculation. For the biochemical assay, next reagents and conditions have been used: 1 × 10⁸ beads M270 Dynabeads^{®} biofunctionalized with a *C. albicans* selective antibody; 5 mM SYTO^{™} 83 Orange Fluorescent Nucleic Acid Stain solution for *Candida* staining; 100 mM glycine solution at pH 12 and 1 M Tris-HCl solution, as elution and conditioning buffers; 5×10⁵ magnetic SERS Tags, with RL1 (4-Mercaptopyridine, 4-MPy) and a selective antibody to C. *albicans;* and 5×10⁵ magnetic SERS tags, with RL2 (4-mercaptobenzoic acid, 4-MBA) and a selective antibody to *S. aureus.* Final measures have been done in a perfusion chamber using around 100 µL of the sample.

- Sample measurement: White light and fluorescence images have been collected with a Nikon Eclipse TS2 microscope, using the ET CY3/TRITC (525 nm filter set) filters. A 100X objective has been preferred in this case, with acquisition times of 500 ms. Raman measurements have been done in a RA816 Renishaw plc equipment using a 785 nm laser and a 50X, LD, objective. SERS mapping was performed at a 1 µm step size. White light images have been also collected with this equipment.

Figure 11 shows a white light and a fluorescence image collected for the blank, where it can be observed the magnetic SERS Tags added in the biochemical assay but no fluorescence due to the absence of analyte.

Figure 12 shows a white light and a fluorescence image collected for the sample, where it can be observed that the magnetic SERS Tags added in the biochemical assay are surrounding the fungi, which fluorescence is also clear.

Figure 13 shows two white light images, one with the SERS signal from the magnetic SERS Tags overlapped. Signal from two different magnetic SERS Tags can be well distinguished. One type has been synthesized with 4-MPy as Raman label and has been biofunctionalized with an antibody selective to *C. albicans,* and the other has been synthesized with 4-MBA as Raman label and biofunctionalized with an antibody selective to *S. aureus.* As it can be seen, the fungus is only surrounded by the magnetic SERS Tags with one type of SERS Tags, 4-MPy, identifying the presence of *C. albicans.*

### Example 9 - Detection and identification of S. aureus

Next example shows the results obtained in an assay with a blood sample which has been inoculated with a known concentration of *S. aureus.* Two different types of SERS Tags have been used, one biofunctionalized with a *C. albicans* selective antibody, and the other with a *S. aureus* selective antibody. Thus, two different Raman labels identify the described SERS Tags.
- Experimental conditions: For the bacteria sample, 10,000 cfu of S. *aureus* has been inoculated to 439 µL of blood, finally diluted to 1 mL. For the biochemical assay, next reagents and conditions have been used: 2 × 10⁸ beads M270 Dynabeads^{®} biofunctionalized with a *S. aureus* selective antibody; 5 mM SYTO^{™} 83 Orange Fluorescent Nucleic Acid Stain solution for bacteria staining; 100 mM glycine solution at pH 12 and 1 M Tris-HCl solution as elution and conditioning buffers; 5×10⁵ magnetic SERS Tags, with RL1 (4-Mercaptopyridine, 4-MPy) and a selective antibody to *C. albicans;* and 5×10⁵magnetic SERS tags, with RL2 (4-mercaptobenzoic acid, 4-MBA) and a selective antibody to *S. aureus.* Final measures have been done in a perfusion chamber using around 100 µL of the sample.
- Sample measurement: White light and fluorescence images have been collected with a Nikon Eclipse TS2 microscope, using the ET CY3/TRITC (525 nm filter set) filters. A 100X objective has been preferred in this case, with acquisition times of 500 ms. Raman measurements have been done in a RA816 Renishaw plc equipment using a 785 nm laser and a 50X, LD, objective. SERS mapping was performed at a 1 µm step size. White light images have been also collected with this equipment.

Figure 14 shows white light images, and the corresponding images with the SERS signal from the magnetic SERS Tags overlapped. Signal from two different magnetic SERS Tags can be well distinguished. One type has been synthesized with 4-MPy as Raman label and has been biofunctionalized with an antibody selective to *C. albicans,* and the other has been synthesized with 4-MBA as Raman label and biofunctionalized with an antibody selective to *S. aureus.* As it can be seen, bacteria are only surrounded by the magnetic SERS Tags with one type of SERS Tags, 4-MBA, identifying the presence of *S. aureus.*

### Example 10. Detection and identification of C. albicans and S. aureus

Next example shows the results obtained in an assay with a blood sample which has been inoculated with a known concentration of *C. albicans* and *S. aureus.* Two different types of SERS Tags have been used, one biofunctionalized with a *C. albicans* selective antibody, and the other with a *S. aureus* selective antibody. Thus, two different Raman labels identify the described SERS Tags.
- Experimental conditions: For the microorganism sample, 10,000 cfu of *C. albicans* and 10,000 cfu of *S. aureus* has been inoculated to 439 µL of blood, finally diluted to 1 mL. For the biochemical assay, next reagents and conditions has been used: 1 × 10⁸ beads M270 Dynabeads^{®} biofunctionalized with a *C. albicans* selective antibody; 2 × 10⁸ beads M270 Dynabeads^{®} biofunctionalized with a *S. aureus* selective antibody; 5 mM SYTO^{™} 83 Orange Fluorescent Nucleic Acid Stain solution for bacteria staining; 100 mM glycine solution at pH 12 and 1 M Tris-HCl solution as elution and conditioning buffers; 5×10⁵ magnetic SERS Tags, with RL1 (4-Mercaptopyridine, 4-MPy) and a selective antibody to *C. albicans;* and 5×10⁵ magnetic SERS tags, with RL2 (4-mercaptobenzoic acid, 4-MBA) and a selective antibody to *S. aureus.* Final measures have been done in a perfusion chamber using around 100 µL of the sample.
- Sample measurement: White light and fluorescence images have been collected with a Nikon Eclipse TS2 microscope, using the ET CY3/TRITC (525 nm filter set) filters. A 100X objective has been preferred in this case, with acquisition times of 500 ms. Raman measurements have been done in a RA816 Renishaw plc equipment using a 785 nm laser and a 50X, LD, objective. SERS mapping was performed at a 1 µm step size. White light images have been also collected with this equipment.

Figure 15 shows white light and fluorescence images collected for the sample, where it can be observed that the magnetic SERS Tags added in the biochemical assay are surrounding the fungi (upper image) and the bacteria (lower image), which fluorescence staining is also clear.

Figure 16 shows white light images, and the corresponding images with the SERS signal from the magnetic SERS Tags overlapped. Signal from two different magnetic SERS Tags can be well distinguished. One type has been synthesized with 4-MPy as Raman label and has been biofunctionalized with an antibody selective to *C. albicans,* and the other has been synthesized with 4-MBA as Raman label and biofunctionalized with an antibody selective to *S. aureus.* As it can be seen, *C. albicans* are labelled with 4-MPy while *S. aureus* is labelled with 4-MBA.

### Example 11. Use of different sizes and material for SERS Tags

Examples from figures 17 to 19 show the results obtained from incubation of *C. albicans* fungi when different sizes (0.45 µm, 2.8 µm or 4.5 µm) of magnetic SERS Tags. Images from figure 20 are related to same assay when using 0.5 µm polystyrene microparticles. In all cases, the employed SERS Tags have 4-MBA as Raman label, are titania coated, and have been biofunctionalized with a *C. albicans* selective antibody. As it can be seen, there is a clear identification of the pathogen. As negative control to check cross-reactivity, same particles were employed in an equivalent assay with *S. aureus,* showing no attachment of the particles to the bacteria.
- Experimental conditions: 100,000 cfu of *C. albicans* fungi were incubated for 1 h at 37°C with 1×10⁶ SERS Tags.
- Sample measurement: White light images have been collected with a Nikon Eclipse TS2 microscope, using the ET CY3/TRITC (525 nm filter set) filters and a 100X oil-immersion objective.

Raman measurements have been done in a RA816 Renishaw plc equipment using a 785 nm laser and a 50X, LD, objective. SERS mapping was performed at a 1 µm step size. White light images have been also collected with this equipment.

### Example 12. Detection of Circulating Tumour Cells by SERS Tags

Figure 21 shows an example of the use of the SERS Tags for cell identification. In this case, AU565 breast cancer cells, which amplifies and overexpresses the HER-2/neu oncogene, have been incubated with 2.8 µm-diameter magnetic SERS Tags biofunctionalized with a selective HER2 antibody. These Tags are labelled with 4-MBA as Raman label, are titania coated, and are dopamine functionalized. As it can be seen in the microscope images, the developed Tags are attached to cells.
- Experimental conditions: 250 AU565 cells in PBS-Tween20^{®} have been incubated with 2.5×10⁵ magnetic SERS Tags for 1 h at 4°C.
- Sample measurement: White light images have been collected with a Nikon Eclipse TS2 microscope, using the ET CY3/TRITC (525 nm filter set) filters and 40x or 100X oil-immersion objectives.

## Claims

1. A surface-enhanced Raman scattering-(SERS)-active reagent, comprising in order:
a) a core of a particle functionalized for binding of element b);
b) a shell of a plurality of nanoparticles of a plasmonic material immobilized on the surface of the particle a) wherein the surface of the nanoparticles is coated with aliphatic, aromatic and/or polymeric molecules or combinations thereof, and at least one Raman label,
c) at least one layer of a coating material covering element b) that interacts with element d); optionally, the layer of a coating material is functionalized with a compound that allows the immobilization of a reagent capable of detecting an analyte; and
d) a reagent immobilized on the coating layer c) capable of detecting an analyte.

2. The SERS active reagent according to claim 1, wherein the particle a) has a size above 0.3 µm, preferably 0.3 to 8 µm, more preferably 0.3 to 4.5 µm, more preferably 0.3-0.8 µm, more preferably 2.5-4.5 µm, more preferably 0.5-1 µm.

3. The SERS active reagent according to any one of claims 1 to 2, wherein the functionalization of particle a) is done by covalent or electrostatic (layer-by-layer) binding, by means of an amino, carboxylic acid, epoxy, sulphonate, phosphate, thiol, chlorosulfonyl, alkene, alkyne and azide functional group, more preferably an amino, carboxylic acid or epoxy functional groups.

4. The SERS active reagent according to any one of claims 1 to 3, wherein the particle has superparamagnetic properties.

5. The SERS active reagent according to any one of aspects 1 to 4, wherein the size of the nanoparticles is 5 to 200 nm, preferably 30-90 nm.

6. The SERS active reagent according to any one of claims 1 to 5, wherein the plasmonic material is selected from the group consisting of gold, silver, copper, palladium, rhodium, aluminum, ruthenium, indium, alkaline, alkaline-earth metals, metals alloys thereof, CuSe, CuTe, silicon and the combinations thereof, particularly silver or gold.

7. The SERS active reagent according to any one of claims 1 to 6, wherein the at least one Raman label is selected from the group consisting of organic dyes, organometallic compounds and aromatic compounds.

8. The SERS active reagent according to any one of claims 1 to 7, wherein the coating material is selected from the group consisting of a metal oxide, a polymer, a protein, a polysaccharide and a lipid.

9. The SERS active reagent according to claim 8, wherein the coating material is a metal oxide, particularly silicon dioxide or titanium dioxide.

10. The SERS active reagent according to any one of claims 1 to 9, wherein the layer c) has a thickness of 1 to 300 nm; preferably from 1 to 120 nm.

11. The SERS active reagent according to any one of claims 1 to 10, wherein the reagent capable of detecting an analyte is selected from the group consisting of an antibody or functional fragments thereof, aptamers, enzymes, nucleic acids, and peptides.

12. A method for detecting and identifying the presence and/or the amount of at least one analyte in a sample, the method comprising:
a) incubating the sample comprising the analyte with a reagent capable of recognizing the analyte, during a time sufficient to allow the formation of a complex between the reagent and the analyte;
b) separating the complex of the reagent and the analyte from the sample;
c) optionally, staining the analyte with a fluorescence label;
d) eluting the analyte from the complex
e) incubating the eluted analyte, with at least one SERS reagent according to any one of claims 1 to 11 during a time sufficient to allow the labelling of the eluted analyte with the SERS reagent; and
f) acquiring an image of the sample with an optical and/or fluorescence microscope and
g) measuring Raman spectroscopy to detect and identify the presence and/or amount of the at least one analyte.

13. The method according to claim 12, wherein the sample is a fluid sample.

14. A method for synthesizing the reagent of any one of claims 1 to 11 comprising the steps of:
a) providing particles functionalized for binding nanoparticles of step b)
b) adding nanoparticles of a plasmonic material and allowing the immobilization of a plurality of nanoparticles on the surface of the particles of step a) to form a cluster, wherein the surface of the nanoparticles is coated with aliphatic, aromatic and/or polymeric molecules or combinations thereof and at least one Raman label;
c) covering the surface of the cluster with at least one layer of a coating material;
d) optionally, functionalizing the coated material with a compound that allows the immobilization of a reagent capable of detecting an analyte; and
e) adding a reagent capable of detecting an analyte to allow the binding of the reagent to the coated cluster.

15. Use of the surface-enhanced Raman scattering-(SERS)-active reagent according to any one of claims 1 to 11 in a method for detecting, identifying and/or quantifying an analyte.
